# EUROPEAN PATENT SPECIFICATION

(11) **EP 0 825 175 B1**
(45) Date of publication and mention of the grant of the patent: **13.06.2001**
(21) Application number: 97112923.4
(22) Date of filing: 28.07.1997
(51) Int. Cl.: C07C 211/63, A01N 33/12

(54) **Quaternary ammonium disinfectant**
Quaternäre Ammoniumverbindungen als Desinfektionsmittel
Désinfectant à l'ammonium quaternaire

(30) Priority: 22.08.1996 JP 22148796; 25.02.1997 JP 4051697
(43) Date of publication of application: 25.02.1998
(73) Proprietor: NICCA CHEMICAL CO., LTD., Fukui-shi Fukui 910 (JP)
(72) Inventor: Kurose, Mikihiko, Yoshida-gun, Fukui (JP); Kameoka, Ikuo, Fukui-shi, Fukui (JP); Makino, Masahiro, Sabae-shi, Fukui (JP)
(74) Representative: Minderop, Ralph H., Dr. rer. nat.

(56) References cited:
- US-A- 2 519 440
- IMAM, T. ET AL: "Bisquaternary ammonium salt. Part 3. Preparation and antimicrobial activity of some new bisquaternary ammonium salts" PHARMAZIE (1983), 38(5), 308-10 CODEN: PHARAT;ISSN: 0031-7144, 1983, XP002047104
- SONG, LI D. ET AL: "Surface Properties, Micellization, and Premicellar Aggregation of Gemini Surfactants with Rigid and Flexible Spacers" LANGMUIR (1996), 12(5), 1149-53 CODEN: LANGD5;ISSN: 0743-7463, 1996, XP002047126
- CHEMICAL ABSTRACTS, vol. 111, no. 22, 27 November 1989 Columbus, Ohio, US; abstract no. 205308, MORIMOTO, KIYOSHI ET AL: "Processing of silver halide color photographic material" XP002047106 & RN: 123603-91-8; RN: 123603-90-7 & JP 01 029 844 A (FUJI PHOTO FILM CO., LTD., JAPAN)
- CHEMICAL ABSTRACTS, vol. 100, no. 8, 20 February 1984 Columbus, Ohio, US; abstract no. 53109, DAIICHI KOGYO SEIYAKU CO., LTD., JAPAN: "Dyeing of acrylic fibers" XP002047107 & RN: 88458-77-9; RN: 88458-76-8 & JP 58 126 383 A (DAIICHI KOGYO SEIYAKU CO., LTD., JAPAN)

## Description

### BACKGROUND OF THE INVENTION

### 1. Field of the Invention

The present invention relates to the use of quaternary ammonium salts as disinfectants.

### 2. Description of the Related Art

Conventional disinfectants include gas type of ethylene oxide, etc., aldehyde type of glutaraldehyde, etc., alcohol type of ethanol, etc., peroxide type of hydrogen peroxide, etc., iodine type of iodine tincture, iodoform, etc., chlorine compound type of hypochlorous acid, chloramine, etc., and phenol type of phenol soap, etc. However, all such disinfectants have had drawbacks including odor, irritation and toxicity, as well as effects on containers and utensils, and therefore it has been necessary to determine their proper use depending on the purpose. Quaternary ammonium salts such as benzalkonium chloride, amphoteric surfactants such as alkyldiamino-ethylglycine hydrochloride and biguanide-based compounds such as chlorhexidine digluconate are used in a variety of different fields because they are odorless and have low toxicity and irritation, minimal effect on containers and utensils, and satisfactory bacterial action. In particular, the wide use of the biguanide-based disinfectant chlorhexidine gluconate for its excellent bacteriocidal effect has led to the discovery of many drug-resistant bacterial strains with resistance to the disinfectant. Future opportunistic infection by such drug-resistant microorganisms has become a risk, as seen in the problem of hospital infection by MRSA (methicillin-resistant *Staphylococcus aureus*). Imam et al. disclose in Pharmazie 38 (1983), pages 308 to 310, α,α-p-xylenebis (decyldimethylammonium) dibromide and its antimicrobial activity.

Yet, despite the more frequent appearance of strains resistant to chlorhexidine digluconate as compared to benzalkonium chloride or alkyldiaminoethylglycine hydrochloride, no superior disinfectant has yet been found.

### SUMMARY OF THE INVENTION

It is an object of the present invention to provide a disinfectant comprising a quaternary ammonium salt compound which has a more excellent bacteriocidal action than benzalkonium chloride, alkyldiamino-ethylglycine hydrochloride or chlorhexidine digluconate and which is suitable as a disinfectant for medical care and working areas.

As a result of diligent research aimed at overcoming the problems described above, the inventors have completed the present invention based on the finding that quaternary ammonium salts with two cationic groups in their compounds have strong bacteriocidal activity.

That is, the present invention proposes to use a quaternary ammonium salt represented by the following general formula (1): 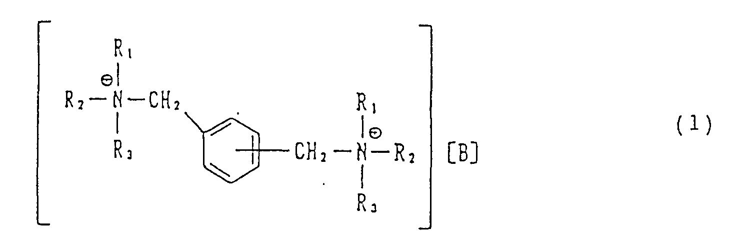 wherein R₁ represents a dodecyl, tridecyl or tetradecyl group 12, R₂ and R₃ each represent an alkyl group of 1-2 carbon atoms, and [B] represents two monovalent inorganic or organic anion groups or one divalent inorganic or organic anion group as disinfectant.

### BRIEF DESCRIPTION OF THE DRAWINGS

Fig. 1 is a ¹H-NMR spectrum of the compound obtained in Example 1.

Fig. 2 is a ¹H-NMR spectrum of the compound obtained in Example 3.

Fig. 3 is a ¹H-NMR spectrum of the compound obtained in Example 27.

Fig. 4 is a ¹H-NMR spectrum of the compound obtained in Example 44.

### DESCRIPTION OF THE PREFERRED EMBODIMENTS

According to the present invention, the inorganic or organic anion group [B] may preferably be a sulfate ion, phosphate ion, phosphite ion, hypophosphite ion, nitrate ion, nitrite ion, chlorate ion, chlorite ion, hypochlorite ion or any of the anion groups represented by the following general formulas (2)-(8).

2[X⁻] (2)

wherein X represents a chlorine atom, bromine atom or iodine atom;

2[R₄COO⁻] (3)

wherein R₄ represents an alkyl group or alkenyl group of 1-7 carbon atoms which may have a hydroxyl group or carbonyl group;

[⁻OCO-R₅-COO⁻] (4)

wherein R₅ represents a direct bond or an alkylene group or alkenylene group of 1-8 carbon atoms which may have a hydroxyl group; 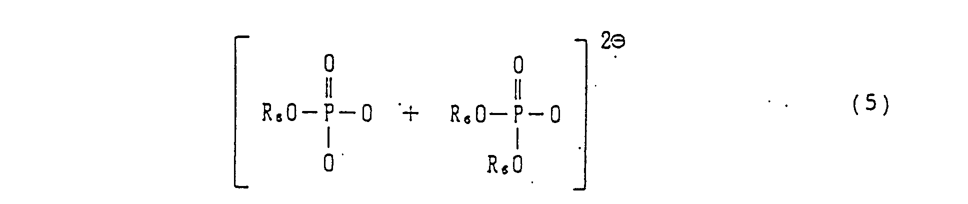 wherein each R₆ represents an alkyl group of 1-8 carbon atoms, and the phosphate ester anion may be a single monoester or diester or a mixture thereof; 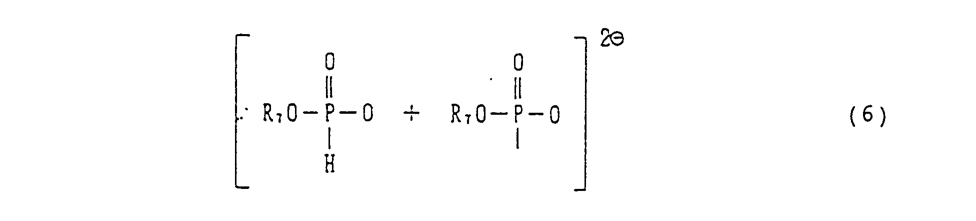 wherein each R₇ represents an alkyl group of 1-8 carbon atoms, and the phosphonate ester anion may be a single monoester or diester or a mixture thereof; 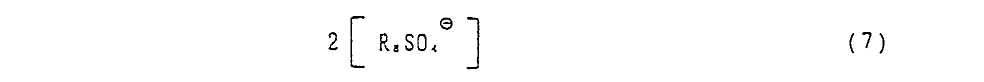 wherein Rₛ represents an alkyl group of 1-2 carbon atoms; 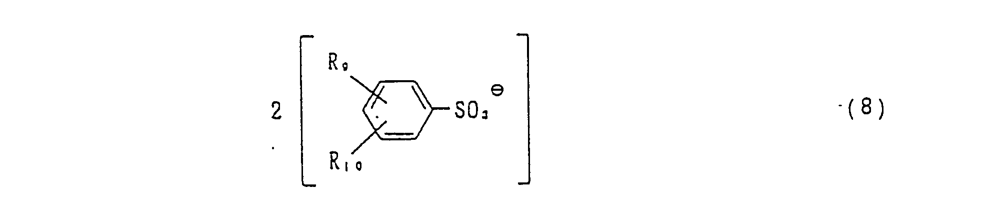 wherein R₉ and R₁₀ each represent a hydrogen atom, methyl group or carboxyl group.

The quaternary ammonium salt represented by general formula (1) above used according to the invention may be produced by reaction of a quaternizing agent of the following general formula (9) and a tertiary amine of the following general formula (10). 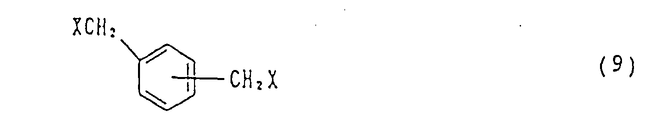 wherein X represents a chlorine atom, bromine atom or iodine atom; 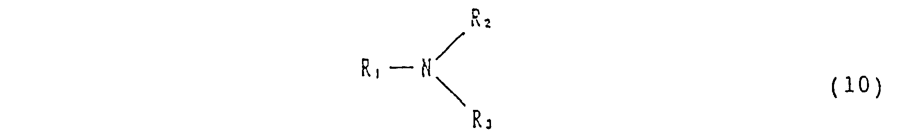 wherein R₁ represents a dodecyl, tridecyl or tetradecyl group 12, and R₂ and R₃ each represent an alkyl group of 1-2 carbon atoms.

As compounds of general formula (9) above there may be mentioned, for example, halogenated compounds such as α,α'-dichloro-p-xylene, α,α'-dichloro-m-xylene, α,α'-dichloro-o-xylene, α,α'-dibromo-p-xylene, α,α'-dibromo-m-xylene, α,α'-dibromo-o-xylene, α,α'-diiodo-p-xylene, α,α'-diiodo-m-xylene and α,α'-diiodo-o-xylene. As compounds of general formula (10) above there may be mentioned, for example, tertiary amines such as N,N-dimethyl-dodecylamine, N,N-dimethyl-tridecylamine, N,N-dimethyl-tetradecylamine, N,N-diethyl-dodecylamine, N,N-diethyl-tridecylamine and N,N-diethyl-tetradecylamine.

The quaternary ammonium salt represented by general formula (1) may be produced by reaction of a tertiary amine represented by the following general formula (11) and a quaternizing agent represented by the following general formula (12). 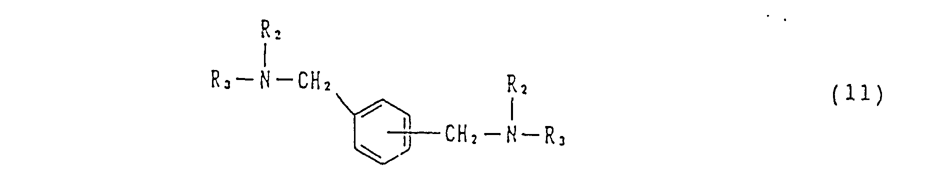 wherein R₂ and R₃ each represent an alkyl group of 1-2 carbon atoms;

R₁-X (12)

wherein R₁ represents a dodecyl, tridecyl or tetradecyl group 12 and X represents a chlorine atom, bromine atom or iodine atom.

As compounds of general formula (11) above there may be mentioned, for example, tertiary amines such as 1,2-bis(N,N-dimethyl-aminomethyl)benzene, 1,3-bis(N,N-dimethyl-aminomethyl)benzene, 1,4-bis(N,N-dimethyl-aminomethyl)benzene, 1,2-bis(N,N-diethyl-aminomethyl)benzene, 1,3-bis(N,N-diethyl-aminomethyl)benzene, 1,4-bis(N,N-diethyl-aminomethyl)benzene. As compounds of general formula (12) above there may be mentioned, for example, alkyl halides such as dodecyl chloride, tridecyl chloride, tetradecyl chloride, dodecyl bromide, tridecyl bromide, tetradecyl bromide, dodecyl iodide, tridecyl iodide and tetradecyl iodide.

The quaternary ammonium salt represented by general formula (1) may also be produced by reaction of a tertiary amine represented by the following general formula (13) and a quaternizing agent represented by the following general formula (14). 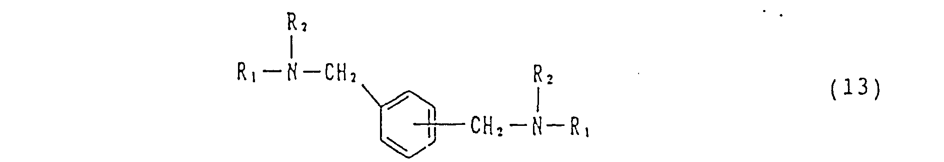 wherein R₁ represents a dodecyl, tridecyl or tetradecyl group 12 and R₂ represents an alkyl group of 1-2 carbon atoms;

R₃-Y (14)

wherein R₃ represents an alkyl group of 1-2 carbon atoms and Y represents a chlorine atom, bromine atom, iodine atom or a group represented by one of the following general formulas (15), (16) or (17). 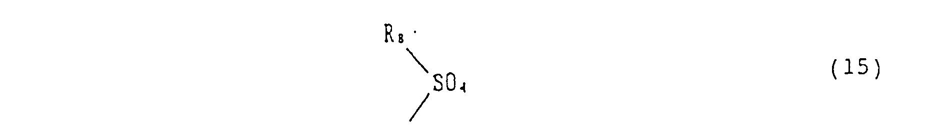 wherein R₈ represents an alkyl group of 1-2 carbon atoms; 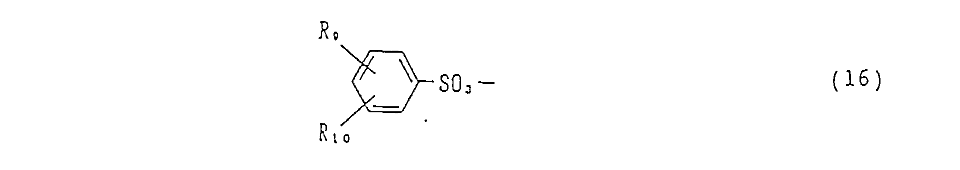 wherein R₉ and R₁₀ each represent a hydrogen atom or methyl group; 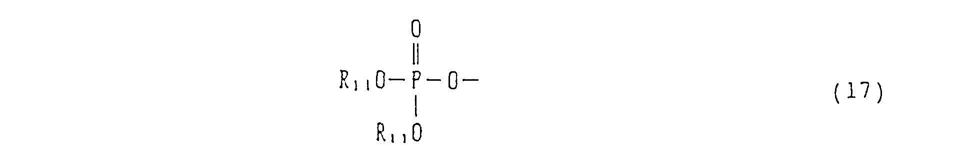 wherein R₁₁ represents an alkyl group of 1-2 carbon atoms.

As compounds represented by general formula (13) above there may be mentioned, for example, tertiary amines such as 1,2-bis(N-dodecyl-N-methylaminomethyl)benzene, 1,2-bis(N-dodecyl-N-ethylaminomethyl)benzene, 1,2-bis(N-tridecyl-N-methylaminomethyl)benzene, 1,2-bis(N-tridecyl-N-ethylaminomethyl)benzene, 1,2-bis(N-tetradecyl-N-methylaminomethyl)benzene, 1,2-bis(N-tetradecyl-N-ethylaminomethyl)benzene, 1,3-bis(N-dodecyl-N-methylaminomethyl)benzene, 1,3-bis(N-dodecyl-N-ethylaminomethyl)benzene, 1,3-bis(N-tridecyl-N-methylaminomethyl)benzene, 1,3-bis(N-tridecyl-N-ethylaminomethyl)benzene, 1,3-bis(N-tetradecyl-N-methylaminomethyl)benzene, 1,3-bis(N-tetradecyl-N-ethylaminomethyl)benzene, 1,4-bis(N-dodecyl-N-methylaminomethyl)benzene, 1,4-bis(N-dodecyl-N-ethylaminomethyl)benzene, 1,4-bis(N-tridecyl-N-methylaminomethyl)benzene, 1,4-bis(N-tridecyl-N-ethylaminomethyl)benzene, 1,4-bis(N-tetradecyl-N-methylaminomethyl)benzene and 1,4-bis(N-tetradecyl-N-ethylaminomethyl)benzene. As compounds of general formula (14) there may be mentioned, for example, alkyl halides such as methyl chloride, ethyl chloride, methyl bromide, ethyl bromide, methyl iodide and ethyl iodide; dialkylsulfuric acids such as dimethylsulfuric acid and diethylsulfuric acid; alkyl sulfonates such as methyl p-toluenesulfonate and ethyl p-toluenesulfonate; and trialkyl phosphates such as trimethyl phosphate and triethyl phosphate.

As reaction solvents for quaternization of these tertiary amines with the quaternizing agent there may preferably be employed water, an alcohol, or a mixed solvent of water and alcohol, and as alcohols there may preferably employed methanol, ethanol, n-propanol, isopropanol, n-butanol, sec-butanol, isobutanol and t-butanol, although they are not limited to these solvents. The reaction temperature will depend on the alkyl chain lengths of the tertiary amine or quaternizing agent, but the reaction usually proceeds rapidly at 80°C or higher.

The quaternary ammonium salt represented by general formula (1) may be produced by ion-exchange of a cationic group-containing quaternary ammonium salt compound of the following general formula (18) with sulfuric acid, phosphoric acid, phosphorous acid, hypophosphorous acid, nitric acid, nitrous acid, chloric acid, chlorous acid, hypochlorous acid or a compound of any of general formulas (19), (20), (21), (22) or (23). 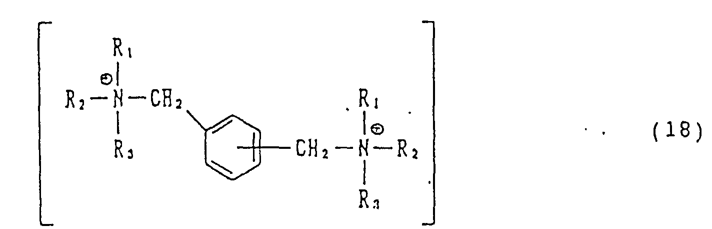 wherein R₁ represents a dodecyl, tridecyl or tetradecyl group 12, and R₇ and R₃ each represent an alkyl group of 1-2 carbon atoms;

R₄-COOH (19)

wherein R₄ represents an alkyl group or alkenyl group of 1-7 carbon atoms which may have a hydroxyl group or carbonyl group;

HOOC-R₅-COOH (20)

wherein R₅ represents a direct bond or an alkylene group or alkenylene group of 1-8 carbon atoms which may have a hydroxyl group; 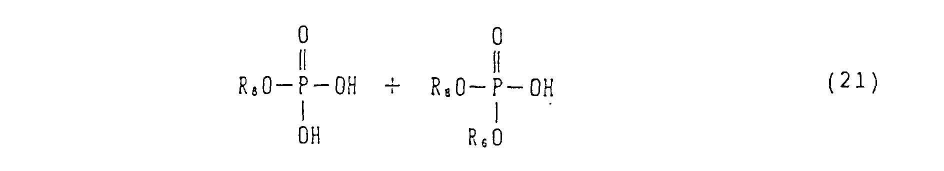 wherein each R₆ represents an alkyl group of 1-8 carbon atoms, and the phosphate ester may be a single monoester or diester or a mixture thereof; 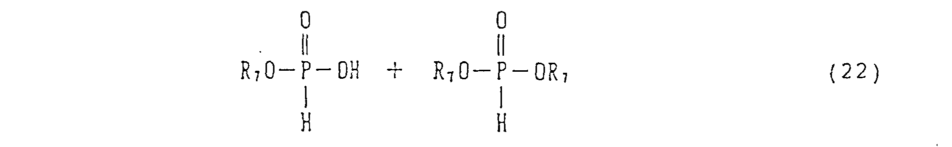 wherein each R₇ represents an alkyl group of 1-8 carbon atoms, and the phosphonate ester may be a single monoester or diester or a mixture thereof; 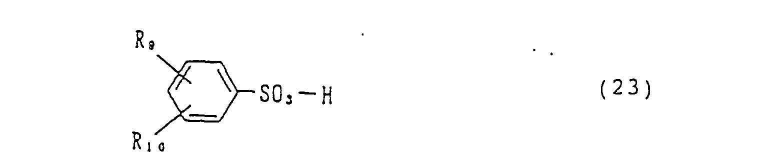 wherein R₉ and R₁₀ each represent a hydrogen atom, methyl group or carboxyl group.

As compounds of general formula (19) above there may be mentioned, for example, carboxylic acids such as acetic acid, propionic acid, butyric acid, isobutyric acid, valeric acid, isovaleric acid, caproic acid, caprylic acid, lactic acid, pyruvic acid, acetoacetic acid, acrylic acid and methacrylic acid, among which as preferred ones there may be mentioned acetic acid, propionic acid, butyric acid, isobutyric acid, lactic acid and acetoacetic acid.

As compounds of general formula (20) there may be mentioned, for example, dicarboxylic acids such as oxalic acid, malonic acid, succinic acid, glutaric acid, adipic acid, pimelic acid, suberic acid, azelaic acid, sebacic acid, tartaric acid, maleic acid and fumaric acid, among which as preferred ones there may be mentioned malonic acid, tartaric acid and maleic acid.

As phosphate ester compounds of general formula (21) there may be mentioned, for example, monoesters and diesters with alkyl groups of 1-8 carbon atoms, and as preferred ones there may be mentioned monoesters and diesters in which the alkyl group is methyl, ethyl, n-propyl, isopropyl, n-butyl, isobutyl, sec-butyl or t-butyl.

As phosphonate ester compounds of general formula (22) there may be mentioned, for example, monoesters and diesters with alkyl groups of 1-8 carbon atoms, and as preferred ones there may be mentioned monoesters and diesters in which the alkyl group is methyl, ethyl, n-propyl, isopropyl, n-butyl, isobutyl, sec-butyl or t-butyl.

As compounds of general formula (23) there may be preferably employed, for example, sulfonic acids such as o-toluenesulfonic acid, m-toluenesulfonic acid, p-toluenesulfonic acid, o-xylene-4-sulfonic acid, m-xylene-4-sulfonic acid, p-xylenesulfonic acid and isophthalylsulfonic acid.

The ion-exchange may be carried out easily by treatment in a column filled with an anion-exchange resin.

The present invention will now be further explained by way of the following examples.

### Example 1

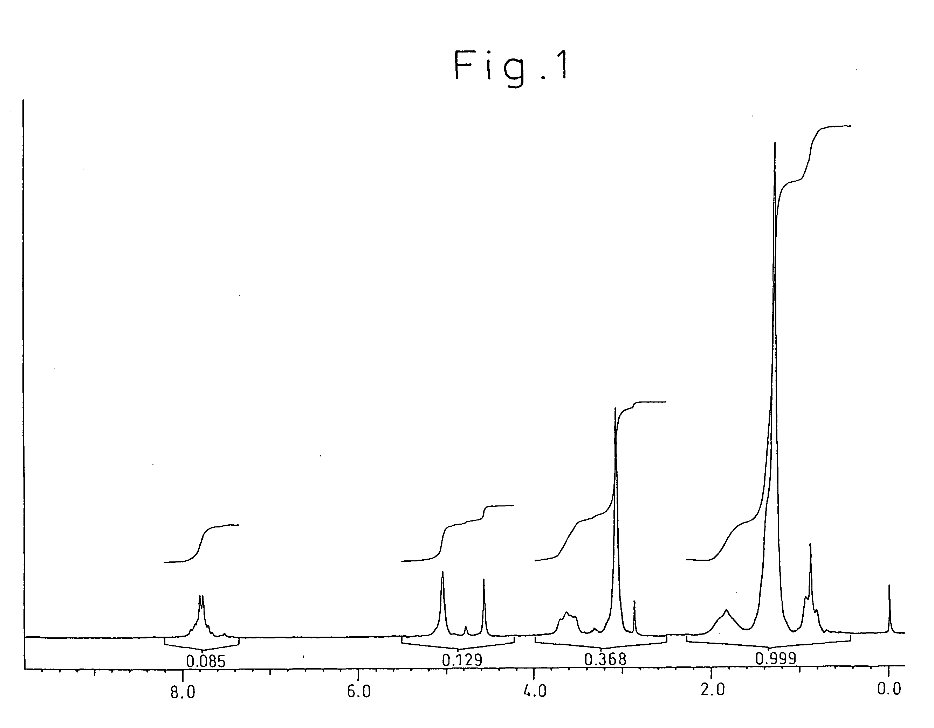

After charging 42.6g of dodecyldimethylamine and 50 ml of water in a 500 ml reaction container the mixture was heated to 75°C. Next, a solution of 17.5g of α,α'-dichloro-o-xylene and 100 ml of isopropanol was slowly added dropwise. After the dropwise addition, a reaction was conducted for one hour while heating to reflux to obtain a transparent homogeneous solution. After maturing for 2 hours, the solution was subjected to steam distillation while slowly adding 200 ml of water to distill off the alcohol portion. After the steam distillation, water was added to the 1,2-bis(N,N-dimethyl-N-dodecylammoniomethyl)benzene dichloride to adjust the concentration to 10 wt%. The results of ¹H-nuclear magnetic resonance spectrum (hereinafter abbreviated to ¹H-NMR. Measuring apparatus: FT-NMR R-1900, product of Hitachi Laboratories) analysis using a 2% heavy methanol solution with a tetramethylsilane indicator (Fig. 1) identified it as the object compound.

### Example 2

After charging 42.6g of dodecyldimethylamine and 50 ml of water in a 500 ml reaction container the mixture was heated to 75°C. Next, a solution of 17.5g of α,α'-dichloro-m-xylene and 100 ml of isopropanol was slowly added dropwise. After the dropwise addition, a reaction was conducted for one hour while heating to reflux to obtain a transparent homogeneous solution. After maturing for 2 hours, the solution was subjected to steam distillation while slowly adding 200 ml of water to distill off the alcohol portion. After the steam distillation, water was added to the 1,3-bis(N,N-dimethyl-N-dodecylammoniomethyl)benzene dichloride product to adjust the concentration to 10 wt%.

### Example 3

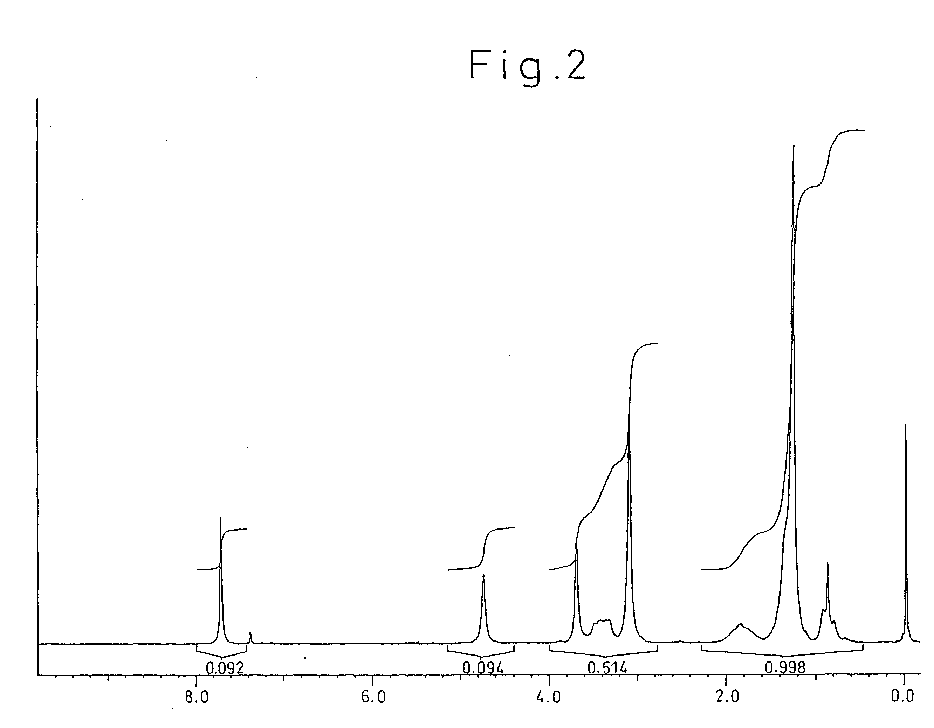

After charging 42.6g of dodecyldimethylamine and 50 ml of water in a 500 ml reaction container the mixture was heated to 75°C. Next, a solution of 17.5g of α,α'-dichloro-p-xylene and 200 ml of isopropanol was slowly added dropwise. After the dropwise addition, a reaction was conducted for one hour while heating to reflux to obtain a transparent homogeneous solution. After maturing for 2 hours, the solution was subjected to steam distillation while slowly adding 300 ml of water to distill off the alcohol portion. After the steam distillation, the purified solution was cooled and the precipitated crystals were filtered off and washed with cold water. Water was added to the obtained 1,4-bis(N,N-dimethyl-N-dodecylammoniomethyl)benzene dichloride crystals to dissolve them, and the concentration was adjusted to 1 wt%. The results of ¹H-NMR analysis (Fig. 2) identified it as the object compound.

### Example 4

After charging 48.2g of tetradecyldimethylamine and 50 ml of water in a 500 ml reaction container the mixture was heated to 75°C. Next, a solution of 17.5g of α,α'-dichloro-o-xylene and 100 ml of isopropanol was slowly added dropwise. After the dropwise addition, a reaction was conducted for one hour while heating to reflux to obtain a transparent homogeneous solution. After maturing for 2 hours, the solution was subjected to steam distillation while slowly adding 200 ml of water to distill off the alcohol portion. After the steam distillation, water was added to the 1,2-bis(N,N-dimethyl-N-tetradecylammoniomethyl)benzene dichloride product to adjust the concentration to 10 wt%.

### Example 5

After charging 48.2g of tetradecyldimethylamine and 50 ml of water in a 500 ml reaction container the mixture was heated to 75°C. Next, a solution of 17.5g of α,α'-dichloro-m-xylene and 100 ml of isopropanol was slowly added dropwise. After the dropwise addition, a reaction was conducted for one hour while heating to reflux to obtain a transparent homogeneous solution. After maturing for 2 hours, the solution was subjected to steam distillation while slowly adding 200 ml of water to distill off the alcohol portion. After the steam distillation, water was added to the 1,3-bis(N,N-dimethyl-N-tetradecylammoniomethyl)benzene dichloride product to adjust the concentration to 5 wt%.

### Example 6

After charging 48.2g of tetradecyldimethylamine and 50 ml of water in a 500 ml reaction container the mixture was heated to 75°C. Next, a solution of 17.5g of α,α'-dichloro-p-xylene and 100 ml of isopropanol was slowly added dropwise. After the dropwise addition, a reaction was conducted for one hour while heating to reflux to obtain a transparent homogeneous solution. After maturing for 2 hours, the solution was subjected to steam distillation while slowly adding 200 ml of water to distill off the alcohol portion. After the steam distillation, the purified solution was cooled and the precipitated crystals were filtered off and washed with cold water. Water was added to the obtained 1,4-bis(N,N-dimethyl-N-tetradecylammoniomethyl)benzene dichloride crystals to dissolve them, and the concentration was adjusted to 1 wt%.

### Example 7

After charging 17.8g of 1,2-bis(N,N-dimethyl-aminomethyl)benzene, 40.9g of dodecyl chloride, 50 ml of water and 200 ml of isopropanol in a 500 ml reaction container, a reaction was conducted for 3 hours while heating to reflux to obtain a transparent homogeneous solution. After maturing for 2 hours, the solution was subjected to steam distillation while slowly adding 300 ml of water to distill off the alcohol portion. After the steam distillation, water was added to the 1,2-bis(N,N-dimethyl-N-dodecylammoniomethyl)benzene dichloride product to adjust the concentration to 10 wt%.

### Example 8

After charging 17.8g of 1,3-bis(N,N-dimethyl-aminomethyl)benzene, 40.9g of dodecyl chloride, 50 ml of water and 200 ml of isopropanol in a 500 ml reaction container, a reaction was conducted for 3 hours while heating to reflux to obtain a transparent homogeneous solution. After maturing for 2 hours, the solution was subjected to steam distillation while slowly adding 300 ml of water to distill off the alcohol portion. After the steam distillation, water was added to the 1,3-bis(N,N-dimethyl-N-dodecylammoniomethyl)benzene dichloride product to adjust the concentration to 5 wt%.

### Example 9

After charging 17.8g of 1,4-bis(N,N-dimethyl-aminomethyl)benzene, 40.9g of dodecyl chloride, 50 ml of water and 200 ml of isopropanol in a 500 ml reaction container, a reaction was conducted for 3 hours while heating to reflux to obtain a transparent homogeneous solution. After maturing for 2 hours, the solution was subjected to steam distillation while slowly adding 300 ml of water to distill off the alcohol portion. After the steam distillation, water was added to the 1,4-bis(N,N-dimethyl-N-dodecylammoniomethyl)benzene dichloride product to adjust the concentration to 1 wt%.

### Example 10

After charging 17.8g of 1,2-bis(N,N-dimethyl-aminomethyl)benzene, 46.5g of tetradecyl chloride, 50 ml of water and 200 ml of isopropanol in a 500 ml reaction container, a reaction was conducted for 3 hours while heating to reflux to obtain a transparent homogeneous solution. After maturing for 3 hours, the solution was subjected to steam distillation while slowly adding 300 ml of water to distill off the alcohol portion. After the steam distillation, water was added to the 1,2-bis(N,N-dimethyl-N-tetradecylammoniomethyl)benzene dichloride product to adjust the concentration to 10 wt%.

### Example 11

After charging 17.8g of 1,3-bis(N,N-dimethyl-aminomethyl)benzene, 46.5g of tetradecyl chloride, 50 ml of water and 200 ml of isopropanol in a 500 ml reaction container, a reaction was conducted for 3 hours while heating to reflux to obtain a transparent homogeneous solution. After maturing for 3 hours, the solution was subjected to steam distillation while slowly adding 300 ml of water to distill off the alcohol portion. After the steam distillation, water was added to the 1,3-bis(N,N-dimethyl-N-tetradecylammoniomethyl)benzene dichloride product to adjust the concentration to 5 wt%.

### Example 12

After charging 17.8g of 1,4-bis(N,N-dimethyl-aminomethyl)benzene, 46.5g of tetradecyl chloride, 50 ml of water and 200 ml of isopropanol in a 500 ml reaction container, a reaction was conducted for 3 hours while heating to reflux to obtain a transparent homogeneous solution. After maturing for 3 hours, the solution was subjected to steam distillation while slowly adding 300 ml of water to distill off the alcohol portion. After the steam distillation, water was added to the 1,4-bis(N,N-dimethyl-N-tetradecylammoniomethyl)benzene dichloride product to adjust the concentration to 1 wt%.

### Example 13

The compound obtained in Example 1 was treated in a column filled with an anion-exchange resin. The treatment solution was adjusted to pH 6.8 ±0.2 with a methyl phosphate ester (monoester/diester = 50/50), and water was added to the 1,2-bis(N,N-dimethyl-N-dodecylammoniomethyl)benzene methyl phosphate ester salt product to adjust the concentration to 1 wt%.

### Example 14

The compound obtained in Example 2 was treated in a column filled with an anion-exchange resin. The treatment solution was adjusted to pH 6.8 ±0.2 with a methyl phosphate ester (monoester/diester = 50/50), and water was added to the 1,3-bis(N,N-dimethyl-N-dodecylammoniomethyl)benzene methyl phosphate ester salt product to adjust the concentration to 1 wt%.

### Example 15

The compound obtained in Example 3 was treated in a column filled with an anion-exchange resin. The treatment solution was adjusted to pH 6.8 ±0.2 with a methyl phosphate ester (monoester/diester = 50/50), and water was added to the 1,4-bis(N,N-dimethyl-N-dodecylammoniomethyl)benzene methyl phosphate ester salt product to adjust the concentration to 1 wt%.

### Example 16

The compound obtained in Example 4 was treated in a column filled with an anion-exchange resin. The treatment solution was adjusted to pH 6.8 ±0.2 with a methyl phosphate ester (monoester/diester = 50/50), and water was added to the 1,2-bis(N,N-dimethyl-N-tetradecylammoniomethyl)benzene methyl phosphate ester salt product to adjust the concentration to 1 wt%.

### Example 17

The compound obtained in Example 5 was treated in a column filled with an anion-exchange resin. The treatment solution was adjusted to pH 6.8 ±0.2 with a methyl phosphate ester (monoester/diester = 50/50), and water was added to the 1,3-bis(N,N-dimethyl-N-tetradecylammoniomethyl)benzene methyl phosphate ester salt product to adjust the concentration to 1 wt%.

### Example 18

The compound obtained in Example 6 was treated in a column filled with an anion-exchange resin. The treatment solution was adjusted to pH 6.8 ±0.2 with a methyl phosphate ester (monoester/diester = 50/50), and water was added to the 1,4-bis(N,N-dimethyl-N-tetradecylammoniomethyl)benzene methyl phosphate ester salt product to adjust the concentration to 1 wt%.

### Example 19

The compound obtained in Example 1 was treated in a column filled with an anion-exchange resin. The treatment solution was adjusted to pH 6.8 ±0.2 with an ethyl phosphate ester (monoester/diester = 50/50), and water was added to the 1,2-bis(N,N-dimethyl-N-dodecylammoniomethyl)benzene ethyl phosphate ester salt product to adjust the concentration to 1 wt%.

### Example 20

The compound obtained in Example 2 was treated in a column filled with an anion-exchange resin. The treatment solution was adjusted to pH 6.8 ±0.2 with an ethyl phosphate ester (monoester/diester = 50/50), and water was added to the 1,3-bis(N,N-dimethyl-N-dodecylammoniomethyl)benzene ethyl phosphate ester salt product to adjust the concentration to 1 wt%.

### Example 21

The compound obtained in Example 3 was treated in a column filled with an anion-exchange resin. The treatment solution was adjusted to pH 6.8 ±0.2 with an ethyl phosphate ester (monoester/diester = 50/50), and water was added to the 1,4-bis(N,N-dimethyl-N-dodecylammoniomethyl)benzene ethyl phosphate ester salt product to adjust the concentration to 1 wt%.

### Example 22

The compound obtained in Example 1 was treated in a column filled with an anion-exchange resin. The treatment solution was adjusted to pH 6.8 ±0.2 with a propyl phosphate ester (monoester/diester = 50/50), and water was added to the 1,2-bis(N,N-dimethyl-N-dodecylammoniomethyl)benzene propyl phosphate ester salt product to adjust the concentration to 1 wt%.

### Example 23

The compound obtained in Example 2 was treated in a column filled with an anion-exchange resin. The treatment solution was adjusted to pH 6.8 ±0.2 with a propyl phosphate ester (monoester/diester = 50/50), and water was added to the 1,3-bis(N,N-dimethyl-N-dodecylammoniomethyl)benzene propyl phosphate ester salt product to adjust the concentration to 1 wt%.

### Example 24

The compound obtained in Example 3 was treated in a column filled with an anion-exchange resin. The treatment solution was adjusted to pH 6.8 ±0.2 with a propyl phosphate ester (monoester/diester = 50/50), and water was added to the 1,4-bis(N,N-dimethyl-N-dodecylammoniomethyl)benzene propyl phosphate ester salt product to adjust the concentration to 1 wt%.

### Example 25

The compound obtained in Example 1 was treated in a column filled with an anion-exchange resin. The treatment solution was adjusted to pH 7.0 ±0.3 with a butyl phosphate ester (monoester/diester = 50/50), and water was added to the 1,2-bis(N,N-dimethyl-N-dodecylammoniomethyl)benzene butyl phosphate ester salt product to adjust the concentration to 1 wt%.

### Example 26

The compound obtained in Example 2 was treated in a column filled with an anion-exchange resin. The treatment solution was adjusted to pH 7.0 ±0.3 with a butyl phosphate ester (monoester/diester = 50/50), and water was added to the 1,3-bis(N,N-dimethyl-N-dodecylammoniomethyl)benzene butyl phosphate ester salt product to adjust the concentration to 1 wt%.

### Example 27

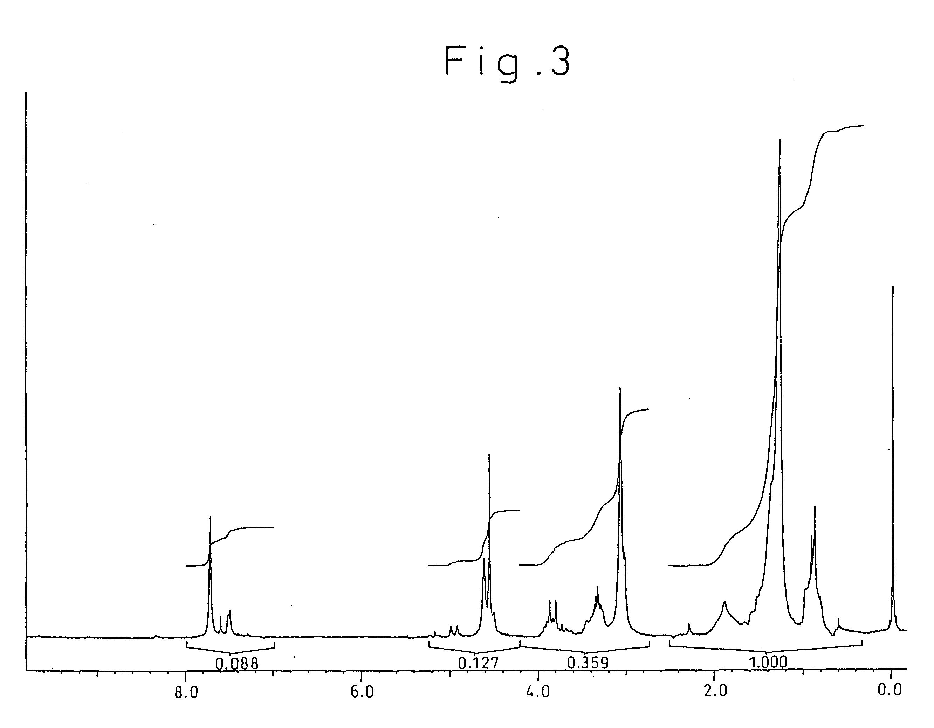

The compound obtained in Example 3 was treated in a column filled with an anion-exchange resin. The treatment solution was adjusted to pH 7.0 ±0.3 with a butyl phosphate ester (monoester/diester = 50/50), and water was added to the 1,4-bis(N,N-dimethyl-N-dodecylammoniomethyl)benzene butyl phosphate ester salt product to adjust the concentration to 1 wt%. The results of ¹H-NMR analysis (Fig. 3) identified it as the object compound.

### Example 28

The compound obtained in Example 1 was treated in a column filled with an anion-exchange resin. The treatment solution was adjusted to pH 7.0 ±0.5 with a hexyl phosphate ester (monoester/diester = 50/50), and water was added to the 1,2-bis(N,N-dimethyl-N-dodecylammoniomethyl)benzene hexyl phosphate ester salt product to adjust the concentration to 1 wt%.

### Example 29

The compound obtained in Example 2 was treated in a column filled with an anion-exchange resin. The treatment solution was adjusted to pH 7.0 ±0.5 with a hexyl phosphate ester (monoester/diester = 50/50), and water was added to the 1,3-bis(N,N-dimethyl-N-dodecylammoniomethyl)benzene hexyl phosphate ester salt product to adjust the concentration to 1 wt%.

### Example 30

The compound obtained in Example 3 was treated in a column filled with an anion-exchange resin. The treatment solution was adjusted to pH 7.0 ±0.5 with a hexyl phosphate ester (monoester/diester = 50/50), and water was added to the 1,4-bis(N,N-dimethyl-N-dodecylammoniomethyl)benzene hexyl phosphate ester salt product to adjust the concentration to 1 wt%.

### Example 31

The compound obtained in Example 1 was treated in a column filled with an anion-exchange resin. The treatment solution was adjusted to pH 7.0 ±0.5 with tartaric acid, and water was added to the 1,2-bis(N,N-dimethyl-N-dodecylammoniomethyl)benzene tartrate salt product to adjust the concentration to 1 wt%.

### Example 32

The compound obtained in Example 1 was treated in a column filled with an anion-exchange resin. The treatment solution was adjusted to pH 7.0 ±0.5 with succinic acid, and water was added to the 1,2-bis(N,N-dimethyl-N-dodecylammoniomethyl)benzene succinate salt product to adjust the concentration to 1 wt%.

### Example 33

The compound obtained in Example 1 was treated in a column filled with an anion-exchange resin. The treatment solution was adjusted to pH 7.0 ±0.5 with malonic acid, and water was added to the 1,2-bis(N,N-dimethyl-N-dodecylammoniomethyl)benzene malonate salt product to adjust the concentration to 1 wt%.

### Example 34

The compound obtained in Example 1 was treated in a column filled with an anion-exchange resin. The treatment solution was adjusted to pH 7.0 ±0.5 with adipic acid, and water was added to the 1,2-bis(N,N-dimethyl-N-dodecylammoniomethyl)benzene adipate salt product to adjust the concentration to 1 wt%.

### Example 35

The compound obtained in Example 1 was treated in a column filled with an anion-exchange resin. The treatment solution was adjusted to pH 7.0 ±0.5 with acetic acid, and water was added to the 1,2-bis(N,N-dimethyl-N-dodecylammoniomethyl)benzene acetate salt product to adjust the concentration to 1 wt%.

### Example 36

The compound obtained in Example 1 was treated in a column filled with an anion-exchange resin. The treatment solution was adjusted to pH 7.0 ±0.5 with lactic acid, and water was added to the 1,2-bis(N,N-dimethyl-N-dodecylammoniomethyl)benzene lactate salt product to adjust the concentration to 1 wt%,

### Example 37

The compound obtained in Example 1 was treated in a column filled with an anion-exchange resin. The treatment solution was adjusted to pH 7.0 ±0.5 with gluconic acid, and water was added to the 1,2-bis(N,N-dimethyl-N-dodecylammoniomethyl)benzene gluconate salt product to adjust the concentration to 1 wt%.

### Example 38

The compound obtained in Example 3 was treated in a column filled with an anion-exchange resin. The treatment solution was adjusted to pH 7.0 ±0.5 with tartaric acid, and water was added to the 1,4-bis(N,N-dimethyl-N-dodecylammoniomethyl)benzene tartrate salt product to adjust the concentration to 1 wt%.

### Example 39

The compound obtained in Example 3 was treated in a column filled with an anion-exchange resin. The treatment solution was adjusted to pH 7.0 ±0.5 with succinic acid, and water was added to the 1,4-bis(N,N-dimethyl-N-dodecylammoniomethyl)benzene succinate salt product to adjust the concentration to 1 wt%.

### Example 40

The compound obtained in Example 3 was treated in a column filled with an anion-exchange resin. The treatment solution was adjusted to pH 7.0 ±0.5 with malonic acid, and water was added to the 1,4-bis(N,N-dimethyl-N-dodecylammoniomethyl)benzene malonate salt product to adjust the concentration to 1 wt%.

### Example 41

The compound obtained in Example 3 was treated in a column filled with an anion-exchange resin. The treatment solution was adjusted to pH 7.0 ±0.5 with adipic acid, and water was added to the 1,4-bis(N,N-dimethyl-N-dodecylammoniomethyl)benzene adipate salt product to adjust the concentration to 1 wt%.

### Example 42

The compound obtained in Example 1 was treated in a column filled with an anion-exchange resin. The treatment solution was adjusted to pH 7.0 ±0.5 with acetic acid, and water was added to the 1,4-bis(N,N-dimethyl-N-dodecylammoniomethyl)benzene acetate salt product to adjust the concentration to 1 wt%.

### Example 43

The compound obtained in Example 3 was treated in a column filled with an anion-exchange resin. The treatment solution was adjusted to pH 7.0 ±0.5 with lactic acid, and water was added to the 1,4-bis(N,N-dimethyl-N-dodecylammoniomethyl)benzene lactate salt product to adjust the concentration to 1 wt%.

### Example 44

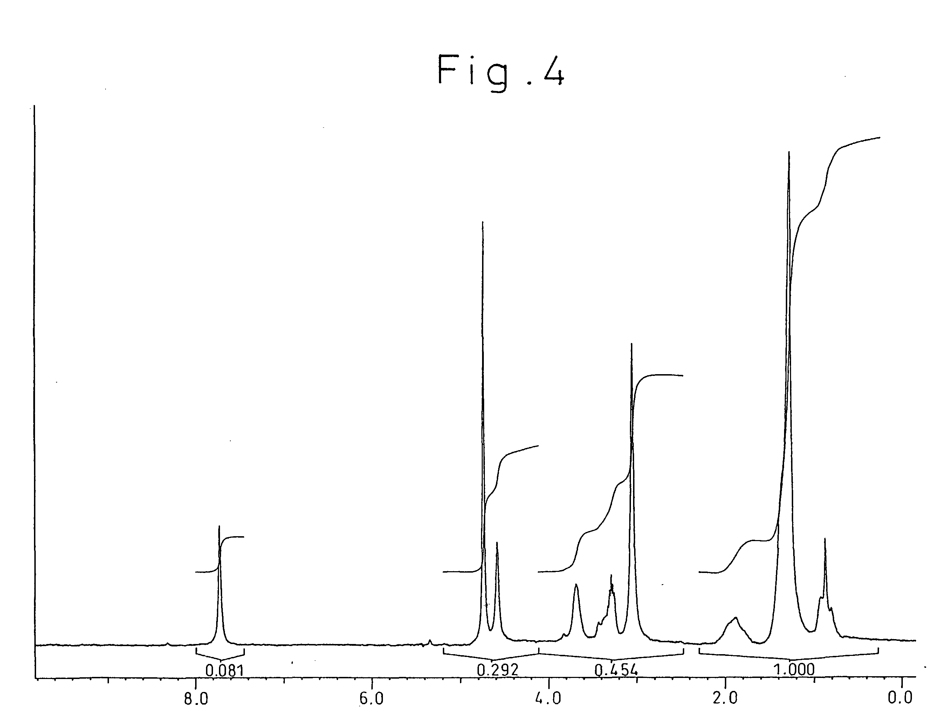

The compound obtained in Example 3 was treated in a column filled with an anion-exchange resin. The treatment solution was adjusted to pH 7.0 ±0.5 with gluconic acid, and water was added to the 1,4-bis(N,N-dimethyl-N-dodecylammoniomethyl)benzene gluconate salt product to adjust the concentration to 1 wt%. The results of ¹H-NMR analysis (Fig. 4) identified it as the object compound.

### Example 45

The compound obtained in Example 2 was treated in a column filled with an anion-exchange resin. The treatment solution was adjusted to pH 7.0 ±0.5 with tartaric acid, and water was added to the 1,3-bis(N,N-dimethyl-N-dodecylammoniomethyl)benzene tartrate salt product to adjust the concentration to 1 wt%.

### Example 46

The compound obtained in Example 2 was treated in a column filled with an anion-exchange resin. The treatment solution was adjusted to pH 7.0 ±0.5 with succinic acid, and water was added to the 1,3-bis(N,N-dimethyl-N-dodecylammoniomethyl)benzene succinate salt product to adjust the concentration to 1 wt%.

### Example 47

The compound obtained in Example 2 was treated in a column filled with an anion-exchange resin. The treatment solution was adjusted to pH 7.0 ±0.5 with malonic acid, and water was added to the 1,3-bis(N,N-dimethyl-N-dodecylammoniomethyl)benzene malonate salt product to adjust the concentration to 1 wt%.

### Example 48

The compound obtained in Example 2 was treated in a column filled with an anion-exchange resin. The treatment solution was adjusted to pH 7.0 ±0.5 with adipic acid, and water was added to the 1,3-bis(N,N-dimethyl-N-dodecylammoniomethyl)benzene adipate salt product to adjust the concentration to 1 wt%.

### Example 49

The compound obtained in Example 2 was treated in a column filled with an anion-exchange resin. The treatment solution was adjusted to pH 7.0 ±0.5 with acetic acid, and water was added to the 1,3-bis(N,N-dimethyl-N-dodecylammoniomethyl)benzene acetate salt product to adjust the concentration to 1 wt%.

### Example 50

The compound obtained in Example 2 was treated in a column filled with an anion-exchange resin. The treatment solution was adjusted to pH 7.0 ±0.5 with lactic acid, and water was added to the 1,3-bis(N,N-dimethyl-N-dodecylammoniomethyl)benzene lactate salt product to adjust the concentration to 1 wt%.

### Example 51

The compound obtained in Example 2 was treated in a column filled with an anion-exchange resin. The treatment solution was adjusted to pH 7.0 ±0.5 with gluconic acid, and water was added to the 1,3-bis(N,N-dimethyl-N-dodecylammoniomethyl)benzene gluconate salt product to adjust the concentration to 1 wt%.

### Example 52

The compound obtained in Example 4 was treated in a column filled with an anion-exchange resin. The treatment solution was adjusted to pH 7.0 ±0.5 with butyl phosphate ester (monoester/diester = 50/50), and water was added to the 1,2-bis(N,N-dimethyl-N-tetradecylammoniomethyl)benzene butyl phosphate ester salt product to adjust the concentration to 1 wt%.

### Example 53

The compound obtained in Example 6 was treated in a column filled with an anion-exchange resin. The treatment solution was adjusted to pH 7.0 ±0.5 with butyl phosphate ester (monoester/diester = 50/50), and water was added to the 1,4-bis(N,N-dimethyl-N-tetradecylammoniomethyl)benzene butyl phosphate ester salt product to adjust the concentration to 1 wt%.

### Example 54

The compound obtained in Example 4 was treated in a column filled with an anion-exchange resin. The treatment solution was adjusted to pH 7.0 ±0.5 with gluconic acid, and water was added to the 1,2-bis(N,N-dimethyl-N-tetradecylammoniomethyl)benzene gluconate ester salt product to adjust the concentration to 1 wt%.

### Example 55

The compound obtained in Example 6 was treated in a column filled with an anion-exchange resin. The treatment solution was adjusted to pH 7.0 ±0.5 with gluconic acid, and water was added to the 1,4-bis(N,N-dimethyl-N-tetradecylammoniomethyl)benzene gluconate ester salt product to adjust the concentration to 1 wt%.

### Example 56

The compound obtained in Example 1 was treated in a column filled with an anion-exchange resin. The treatment solution was adjusted to pH 7.0 ±0.2 with sulfuric acid, and water was added to the 1,2-bis(N,N-dimethyl-N-dodecylammoniomethyl)benzene sulfate salt product to adjust the concentration to 1 wt%.

### Example 57

The compound obtained in Example 1 was treated in a column filled with an anion-exchange resin. The treatment solution was adjusted to pH 7.0 ±0.2 with nitric acid, and water was added to the 1,2-bis(N,N-dimethyl-N-dodecylammoniomethyl)benzene nitrate salt product to adjust the concentration to 1 wt%.

### Example 58

The compound obtained in Example 1 was treated in a column filled with an anion-exchange resin. The treatment solution was adjusted to pH 7.0 ±0.2 with p-toluenesulfonic acid, and water was added to the 1,2-bis(N,N-dimethyl-N-dodecylammoniomethyl)benzene p-toluenesulfonate salt product to adjust the concentration to 1 wt%.
Comparative Example 1
Benzalkonium chloride.
Comparative Example 2
Chlorhexidine digluconate

### Evaluation method

The minimum bacteriocidal concentration (MBC) of the non-volatile portions of the compounds of Examples 1 to 71 and Comparative Examples 1 and 2 were measured according to the Standards of the Japan Chemotherapy Institute, using 2 strains of methicillin-resistant *Staphylococcus aureus* (*S*. *aureus* (MRSA)), 2 strains of *Staphylococcus aureus* (*S*. *aureus),* 3 strains of *Escherichia coli* (*E*. *coli*), 3 strains of *Pseudomonas aeruginosa* (*P*. *aeruginosa),* 2 strains of *Klebsiella pneumoniae* (*K*. *pneumoniae),* 2 strains of *Serratia marcescens* (*S*. *marcescens*) and 2 strains of *Salmonella enteritidis* (*S*. *enteritidis*), and the bacteriocidal effects were compared. The results are shown in Tables 1 to 18.

**Table 1**

| Bacteria strain | MBC (µg/ml) | | | |
|---|---|---|---|---|
| | Comp.Ex.1 | Comp.Ex.2 | Example 1 | Example 2 |
| *S. aureus* (MRSA) A | 50 | 25 | 2.5 | 2.5 |
| *S. aureus* (MRSA) B | 50 | 25 | 2.5 | 2.5 |
| *S. aureus* A | 25 | 25 | 2.5 | 2.5 |
| *S*. *aureus* B | 25 | 50 | 2.5 | 2.5 |
| *E. coli* A | 25 | 5 | 2.5 | 2.5 |
| *E. coli* B | 25 | 10 | 2.5 | 2.5 |
| *E. coli* C | 25 | 10 | 2.5 | 2.5 |
| *P. aeruginosa* A | 25 | 25 | 2.5 | 2.5 |
| *P. aeruginosa* B | 50 | 25 | 2.5 | 2.5 |
| *P. aeruginosa* C | 50 | 100 | 2.5 | 2.5 |
| *K. pneumoniae* A | 50 | 5 | 5 | 5 |
| *K*. *pneumoniae* B | 50 | 5 | 5 | 5 |
| *S. marcescens* A | 25 | 25 | 5 | 5 |
| *S. marcescens* B | 25 | 10 | 5 | 5 |
| *S. enteritidis* A | 25 | 5 | 5 | 5 |
| *S. enteritidis* B | 25 | 5 | 5 | 5 |

**Table 2**

| Bacteria strain | MBC (µg/ml) | | | |
|---|---|---|---|---|
| | Example 3 | Example 4 | Example 5 | Example 6 |
| *S*. *aureus* (MRSA) A | 2.5 | 10 | 5 | 10 |
| *S. aureus* (MRSA) B | 2.5 | 5 | 10 | 5 |
| *S. aureus* A | 2.5 | 5 | 5 | 5 |
| *S. aureus* B | 2.5 | 5 | 5 | 5 |
| *E*. *coli* A | 2.5 | 5 | 5 | 5 |
| *E*. *coli* B | 2.5 | 5 | 10 | 5 |
| *E. coli* C | 2.5 | 10 | 5 | 10 |
| *P. aeruginosa* A | 2.5 | 25 | 25 | 25 |
| *P. aeruginosa* B | 2.5 | 5 | 5 | 5 |
| *P. aeruginosa* C | 2.5 | 5 | 5 | 5 |
| *K. pneumoniae* A | 5 | 25 | 10 | 25 |
| *K*. *pneumoniae* B | 5 | 10 | 25 | 10 |
| *S. marcescens* A | 5 | 10 | 10 | 10 |
| *S. marcescens B* | 5 | 10 | 10 | 10 |
| *S. enteritidis* A | 5 | 5 | 10 | 10 |
| *S. enteritidis* B | 5 | 5 | 10 | 10 |

**Table 3**

| Bacteria strain | MBC (µg/ml) | |
|---|---|---|
| | Example 7 | Example 8 |
| *S*. *aureus* (MRSA) A | 2.5 | 2.5 |
| *S*. *aureus* (MRSA) B | 2.5 | 2.5 |
| *S*. *aureus* A | 2.5 | 2.5 |
| *S*. *aureus* B | 2.5 | 2.5 |
| *E. coli* A | 2.5 | 2.5 |
| *E. coli* B | 2.5 | 2.5 |
| *E. coli* C | 2.5 | 2.5 |
| *P. aeruginosa* A | 2.5 | 2.5 |
| *P. aeruginosa* B | 2.5 | 2.5 |
| *P. aeruginosa C* | 2.5 | 2.5 |
| *K. pneumoniae* A | 5 | 5 |
| *K. pneumoniae* B | 5 | 5 |
| *S. marcescens* A | 5 | 5 |
| *S. marcescens* B | 5 | 5 |
| *S. enteritidis* A | 5 | 5 |
| *S. enteritidis* B | 5 | 5 |

**Table 4**

| Bacteria strain | MBC (µg/ml) | | | |
|---|---|---|---|---|
| | Example 9 | Example 10 | Example 11 | Example 12 |
| *S. aureus* (MRSA) A | 2.5 | 10 | 10 | 10 |
| *S. aureus* (MRSA) B | 2.5 | 10 | 10 | 10 |
| *S. aureus* A | 2.5 | 5 | 5 | 5 |
| *S*. *aureus* B | 2.5 | 5 | 5 | 5 |
| *E. coli* A | 2.5 | 5 | 5 | 5 |
| *E. coli* B | 2.5 | 10 | 10 | 10 |
| *E. coli C* | 2.5 | 5 | 5 | 5 |
| *P. aeruginosa* A | 2.5 | 5 | 5 | 5 |
| *P. aeruginosa* B | 2.5 | 25 | 25 | 25 |
| *P. aeruginosa C* | 2.5 | 5 | 5 | 5 |
| *K. pneumoniae* A | 5 | 25 | 25 | 25 |
| *K. pneumoniae* B | 5 | 10 | 10 | 10 |
| *S. marcescens* A | 5 | 10 | 10 | 10 |
| *S. marcescens* B | 5 | 10 | 10 | 10 |
| *S. enteritidis* A | 5 | 10 | 10 | 10 |
| *S. enteritidis* B | 5 | 10 | 10 | 10 |

**Table 5**

| Bacteria strain | MBC (µg/ml) |
|---|---|
| | Example 13 |
| *S. aureus* (MRSA) A | 2.5 |
| *S. aureus* (MRSA) B | 2.5 |
| *S*. *aureus* A | 2.5 |
| *S. aureus* B | 2.5 |
| *E. coli* A | 2.5 |
| *E. coli* B | 2.5 |
| *E. coli* C | 2.5 |
| *P. aeruginosa* A | 2.5 |
| *P. aeruginosa* B | 2.5 |
| *P. aeruginosa* C | 2.5 |
| *K. pneumoniae* A | 5 |
| *K*. *pneumoniae* B | 5 |
| *S. marcescens* A | 5 |
| *S. marcescens* B | 5 |
| *S. enteritidis* A | 5 |
| *S. enteritidis* B | 5 |

**Table 6**

| Bacteria strain | MBC (µg/ml) | | | |
|---|---|---|---|---|
| | Example 14 | Example 15 | Example 16 | Example 17 |
| *S*. *aureus* (MRSA) A | 2.5 | 2.5 | 10 | 10 |
| *S*. *aureus* (MRSA) B | 2.5 | 2.5 | 10 | 10 |
| *S*. *aureus* A | 2.5 | 2.5 | 5 | 5 |
| *S*. *aureus* B | 2.5 | 2.5 | 5 | 5 |
| *E*. *coli* A | 2.5 | 2.5 | 5 | 5 |
| *E. coli* B | 2.5 | 2.5 | 10 | 10 |
| *E*. *coli C* | 2.5 | 2.5 | 5 | 5 |
| *P*. *aeruginosa* A | 2.5 | 2.5 | 5 | 5 |
| *P*. *aeruginosa* B | 2.5 | 2.5 | 25 | 25 |
| *P*. *aeruginosa C* | 2.5 | 2.5 | 5 | 5 |
| *K*. *pneumoniae* A | 5 | 5 | 25 | 25 |
| *K. pneumoniae* B | 5 | 5 | 10 | 10 |
| *S*. *marcescens* A | 5 | 5 | 10 | 10 |
| *S. marcescens* B | 5 | 5 | 10 | 10 |
| *S. enteritidis* A | 5 | 5 | 10 | 10 |
| *S. enteritidis* B | 5 | 5 | 10 | 10 |

**Table 7**

| Bacteria strain | MBC (µg/ml) | | | |
|---|---|---|---|---|
| | Example 18 | Example 19 | Example 29 | Example 21 |
| *S*. *aureus* (MRSA) A | 10 | 2.5 | 2.5 | 2.5 |
| *S*. *aureus* (MRSA) B | 10 | 2.5 | 2.5 | 2.5 |
| *S*. *aureus* A | 5 | 2.5 | 2.5 | 2.5 |
| *S. aureus B* | 5 | 5 | 2.5 | 5 |
| *E*. *coli* A | 5 | 2.5 | 2.5 | 2.5 |
| *E*. *coli* B | 10 | 2.5 | 2.5 | 2.5 |
| *E. coli* C | 5 | 2.5 | 2.5 | 2.5 |
| *P*. *aeruginosa* A | 5 | 5 | 5 | 5 |
| *P*. *aeruginosa* B | 25 | 5 | 5 | 5 |
| *P*. *aeruginosa* C | 5 | 5 | 5 | 5 |
| *K*. *pneumoniae* A | 25 | 5 | 5 | 5 |
| *K*. *pneumoniae* B | 10 | 5 | 5 | 5 |
| *S*. *marcescens* A | 10 | 5 | 5 | 5 |
| *S*. *marcescens* B | 10 | 5 | 5 | 5 |
| *S*. *enteritidis* A | 10 | 5 | 5 | 5 |
| *S*. *enteritidis* B | 10 | 5 | 5 | 5 |

**Table 8**

| Bacteria strain | MBC (µg/ml) | | | |
|---|---|---|---|---|
| | Example 22 | Example 23 | Example 24 | Example 25 |
| *S*. *aureus* (MRSA) A | 2.5 | 2.5 | 2.5 | 2.5 |
| *S*. *aureus* (MRSA) B | 2.5 | 2.5 | 2.5 | 2.5 |
| *S*. *aureus* A | 5 | 2.5 | 5 | 5 |
| *S*. *aureus* B | 5 | 2.5 | 5 | 5 |
| *E*. *coli* A | 2.5 | 2.5 | 5 | 2.5 |
| *E. coli* B | 2.5 | 2.5 | 5 | 2.5 |
| *E*. *coli* C | 2.5 | 2.5 | 5 | 2.5 |
| *P*. *aeruginosa* A | 5 | 5 | 5 | 5 |
| *P. aeruginosa* B | 5 | 5 | 5 | 5 |
| *P. aeruginosa* C | 5 | 5 | 5 | 5 |
| *K*. *pneumoniae* A | 5 | 5 | 5 | 5 |
| *K. pneumoniae* B | 5 | 5 | 5 | 5 |
| *S*. *marcescens* A | 5 | 5 | 5 | 5 |
| *S. marcescens* B | 5 | 5 | 5 | 5 |
| *S. enteritidis* A | 5 | 5 | 5 | 5 |
| *S. enteritidis B* | 5 | 5 | 5 | 5 |

**Table 9**

| Bacteria strain | MBC (µg/ml) | | | |
|---|---|---|---|---|
| | Example 26 | Example 27 | Example 28 | Example 29 |
| *S*. *aureus* (MRSA) A | 2.5 | 2.5 | 2.5 | 2.5 |
| *S*. *aureus* (MRSA) B | 2.5 | 2.5 | 2.5 | 2.5 |
| *S*. *aureus* A | 2.5 | 2.5 | 5 | 2.5 |
| *S*. *aureus* B | 2.5 | 2.5 | 5 | 2.5 |
| *E*. *coli* A | 2.5 | 5 | 2.5 | 2.5 |
| *E*. *coli B* | 2.5 | 5 | 2.5 | 2.5 |
| *E*. *coli* C | 2.5 | 5 | 2.5 | 2.5 |
| *P*. *aeruginosa* A | 5 | 5 | 5 | 5 |
| *P*. *aeruginosa B* | 5 | 5 | 5 | 5 |
| *P*. *aeruginosa* C | 5 | 5 | 5 | 5 |
| *K*. *pneumoniae* A | 2.5 | 5 | 5 | 5 |
| *K*. *pneumoniae B* | 2.5 | 5 | 5 | 5 |
| *S. marcescens* A | 2.5 | 5 | 5 | 5 |
| *S*. *marcescens* B | 2.5 | 5 | 5 | 5 |
| *S*. *enteritidis* A | 5 | 5 | 5 | 5 |
| *S*. *enteritidis* B | 5 | 5 | 5 | 5 |

**Table 10**

| Bacteria strain | MBC (µg/ml) | | | |
|---|---|---|---|---|
| | Example 30 | Example 31 | Example 32 | Example 33 |
| *S*. *aureus* (MRSA) A | 2.5 | 2.5 | 5 | 2.5 |
| *S*. *aureus* (MRSA) B | 2.5 | 2.5 | 5 | 2.5 |
| *S*. *aureus* A | 2.5 | 5 | 5 | 2.5 |
| *S*. *aureus* B | 2.5 | 5 | 5 | 2.5 |
| *E. coli* A | 5 | 5 | 5 | 2.5 |
| *E. coli B* | 5 | 5 | 5 | 2.5 |
| *E. coli* C | 5 | 5 | 5 | 2.5 |
| *P. aeruginosa* A | 5 | 10 | 10 | 2.5 |
| *P. aeruginosa* B | 5 | 10 | 10 | 5 |
| *P. aeruginosa* C | 5 | 10 | 10 | 5 |
| *K. pneumoniae* A | 5 | 5 | 5 | 5 |
| *K. pneumoniae* B | 5 | 5 | 5 | 2.5 |
| *S. marcescens* A | 5 | 10 | 10 | 5 |
| *S*. *marcescens* B | 5 | 10 | 10 | 5 |
| *S*. *enteritidis* A | 5 | 10 | 10 | 5 |
| *S*. *enteritidis* B | 5 | 10 | 10 | 5 |

**Table 11**

| Bacteria strain | MBC (µg/ml) | | | |
|---|---|---|---|---|
| | Example 34 | Example 35 | Example 36 | Example 37 |
| *S*. aureus (MRSA) A | 2.5 | 2.5 | 2.5 | 2.5 |
| *S*. *aureus* (MRSA) B | 2.5 | 2.5 | 2.5 | 2.5 |
| *S*. *aureus* A | 2.5 | 2.5 | 2.5 | 2.5 |
| *S*. *aureus* B | 2.5 | 2.5 | 2.5 | 2.5 |
| *E. coli* A | 2.5 | 2.5 | 2.5 | 5 |
| *E. coli* B | 2.5 | 2.5 | 2.5 | 5 |
| *E. coli* C | 2.5 | 2.5 | 2.5 | 5 |
| *P. aeruginosa* A | 2.5 | 5 | 5 | 5 |
| *P. aeruginosa* B | 5 | 5 | 5 | 5 |
| *P. aeruginosa* C | 5 | 5 | 5 | 5 |
| *K. pneumoniae* A | 2.5 | 2.5 | 2.5 | 2.5 |
| *K. pneumoniae* B | 2.5 | 2.5 | 2.5 | 2.5 |
| *S. marcescens* A | 5 | 5 | 5 | 5 |
| *S. marcescens* B | 5 | 5 | 5 | 5 |
| *S. enteritidis* A | 5 | 5 | 5 | 2.5 |
| *S. enteritidis* B | 5 | 5 | 5 | 2.5 |

**Table 12**

| Bacteria strain | MBC (µg/ml) | | | |
|---|---|---|---|---|
| | Example 38 | Example 39 | Example 40 | Example 41 |
| *S*. *aureus* (MRSA) A | 2.5 | 5 | 2.5 | 2.5 |
| *S*. *aureus* (MRSA) B | 2.5 | 5 | 2.5 | 2.5 |
| *S*. *aureus* A | 5 | 5 | 2.5 | 2.5 |
| *S. aureus* B | 5 | 5 | 2.5 | 2.5 |
| *E. coli* A | 5 | 5 | 2.5 | 2.5 |
| *E. coli* B | 5 | 5 | 2.5 | 2.5 |
| *E. coli* C | 5 | 5 | 2.5 | 2.5 |
| *P. aeruginosa* A | 10 | 10 | 2.5 | 2.5 |
| *P. aeruginosa* B | 10 | 10 | 5 | 5 |
| *P. aeruginosa* C | 10 | 10 | 5 | 5 |
| *K*. *pneumoniae* A | 5 | 5 | 5 | 2.5 |
| *K. pneumoniae* B | 5 | 5 | 2.5 | 2.5 |
| *S*. *marcescens* A | 10 | 10 | 5 | 5 |
| *S*. *marcescens* B | 10 | 10 | 5 | 5 |
| *S. enteritidis* A | 10 | 10 | 5 | 5 |
| *S*. *enteritidis* B | 10 | 10 | 5 | 5 |

**Table 13**

| Bacteria strain | MBC (µg/ml) | | | |
|---|---|---|---|---|
| | Example 42 | Example 43 | Example 44 | Example 45 |
| *S. aureus* (MRSA) A | 2.5 | 2.5 | 2.5 | 2.5 |
| *S. aureus* (MRSA) B | 2.5 | 2.5 | 2.5 | 2.5 |
| *S. aureus* A | 2.5 | 2.5 | 5 | 5 |
| *S. aureus B* | 2.5 | 2.5 | 5 | 5 |
| *E*. *coli* A | 2.5 | 2.5 | 5 | 5 |
| *E*. *coli* B | 2.5 | 2.5 | 5 | 5 |
| *E*. *coli* C | 2.5 | 2.5 | 5 | 5 |
| *P*. *aeruginosa* A | 5 | 5 | 5 | 10 |
| *P*. *aeruginosa* B | 5 | 5 | 5 | 10 |
| *P*. *aeruginosa* C | 5 | 5 | 5 | 10 |
| *K*. *pneumoniae* A | 2.5 | 2.5 | 10 | 5 |
| *K*. *pneumoniae B* | 2.5 | 2.5 | 5 | 5 |
| *S. marcescens* A | 5 | 5 | 5 | 10 |
| *S. marcescens* B | 5 | 5 | 5 | 10 |
| *S. enteritidis* A | 5 | 5 | 2.5 | 10 |
| *S. enteritidis* B | 5 | 5 | 2.5 | 10 |

**Table 14**

| Bacteria strain | MBC (µg/ml) | | | |
|---|---|---|---|---|
| | Example 46 | Example 47 | Example 48 | Example 49 |
| *S*. *aureus* (MRSA) A | 5 | 2.5 | 2.5 | 2.5 |
| *S*. *aureus* (MRSA) B | 5 | 2.5 | 2.5 | 2.5 |
| *S*. *aureus* A | 5 | 2.5 | 2.5 | 2.5 |
| *S*. *aureus* B | 5 | 2.5 | 2.5 | 2.5 |
| *E. coli* A | 5 | 2.5 | 2.5 | 2.5 |
| *E. coli* B | 5 | 2.5 | 2.5 | 2.5 |
| *E. coli* C | 5 | 2.5 | 2.5 | 2.5 |
| *P*. *aeruginosa* A | 10 | 2.5 | 2.5 | 5 |
| *P*. *aeruginosa* B | 10 | 5 | 5 | 5 |
| *P*. *aeruginosa* C | 10 | 5 | 5 | 5 |
| *K*. *pneumoniae* A | 5 | 5 | 2.5 | 2.5 |
| *K*. *pneumoniae* B | 5 | 2.5 | 2.5 | 2.5 |
| *S*. *marcescens* A | 10 | 5 | 5 | 5 |
| *S*. *marcescens* B | 10 | 5 | 5 | 5 |
| *S*. *enteritidis* A | 10 | 5 | 5 | 5 |
| *S*. *enteritidis* B | 10 | 5 | 5 | 5 |

**Table 15**

| Bacteria strain | MBC (µg/ml) | |
|---|---|---|
| | Example 50 | Example 51 |
| *S*. *aureus* (MRSA) A | 2.5 | 2.5 |
| *S*. *aureus* (MRSA) B | 2.5 | 5 |
| *S*. *aureus* A | 2.5 | 5 |
| *S*. *aureus* B | 2.5 | 2.5 |
| *E. coli* A | 2.5 | 2.5 |
| *E. coli* B | 2.5 | 2.5 |
| *E. coli* C | 2.5 | 2.5 |
| *P. aeruginosa* A | 2.5 | 2.5 |
| *P. aeruginosa* B | 5 | 2.5 |
| *P. aeruginosa* C | 5 | 5 |
| *K. pneumoniae* A | 2.5 | 5 |
| *K. pneumoniae* B | 2.5 | 5 |
| *S. marcescens* A | 5 | 2.5 |
| *S. marcescens* B | 5 | 2.5 |
| *S*. *enteritidis* A | 5 | 2.5 |
| *S*. *enteritidis* B | 5 | 2.5 |

**Table 16**

| Bacteria strain | MBC (µg/ml) | |
|---|---|---|
| | Example 52 | Example 53 |
| *S*. *aureus* (MRSA) A | 5 | 5 |
| *S*. *aureus* (MRSA) B | 5 | 5 |
| *S*. *aureus* A | 5 | 5 |
| *S*. *aureus* B | 10 | 10 |
| *E. coli* A | 5 | 5 |
| *E. coli* B | 5 | 5 |
| *E. coli* C | 5 | 5 |
| *P. aeruginosa* A | 10 | 25 |
| *P. aeruginosa* B | 10 | 5 |
| *P. aeruginosa* C | 5 | 10 |
| *K. pneumoniae* A | 10 | 10 |
| *K. pneumoniae* B | 10 | 10 |
| *S*. *marcescens* A | 25 | 25 |
| *S*. *marcescens* B | 25 | 25 |
| *S. enteritidis* A | 10 | 25 |
| *S*. *enteritidis* B | 10 | 25 |

**Table 17**

| Bacteria strain | MBC (µg/ml) | | | |
|---|---|---|---|---|
| | Example 54 | Example 55 | Example 56 | Example 57 |
| *S. aureus* (MRSA) A | 5 | 5 | 2.5 | 2.5 |
| *S. aureus* (MRSA) B | 5 | 5 | 2.5 | 2.5 |
| *S. aureus* A | 5 | 5 | 2.5 | 2.5 |
| *S. aureus* B | 5 | 5 | 5 | 5 |
| *E. coli* A | 5 | 5 | 2.5 | 2.5 |
| *E. coli* B | 5 | 5 | 2.5 | 2.5 |
| *E. coli* C | 5 | 5 | 2.5 | 2.5 |
| *P. aeruginosa* A | 10 | 25 | 5 | 5 |
| *P*. *aeruginosa* B | 10 | 10 | 5 | 5 |
| *P. aeruginosa* C | 10 | 10 | 5 | 5 |
| *K. pneumoniae* A | 10 | 10 | 5 | 5 |
| *K. pneumoniae B* | 10 | 10 | 5 | 5 |
| *S. marcescens* A | 25 | 25 | 5 | 5 |
| *S. marcescens* B | 25 | 25 | 5 | 5 |
| *S. enteritidis* A | 25 | 25 | 5 | 5 |
| *S. enteritidis* B | 25 | 25 | 5 | 5 |

**Table 18**

| Bacteria strain | MBC (µg/ml) |
|---|---|
| | Example 58 |
| *S. aureus* (MRSA) A | 5 |
| *S. aureus* (MRSA) B | 5 |
| *S*. *aureus* A | 5 |
| *S*. *aureus* B | 5 |
| *E*. *coli* A | 5 |
| *E*. *coli B* | 5 |
| *E*. *coli* C | 5 |
| *P*. *aeruginosa* A | 10 |
| *P*. *aeruginosa* B | 10 |
| *P*. *aeruginosa* C | 10 |
| *K*. *pneumoniae* A | 5 |
| *K*. *pneumoniae* B | 5 |
| *S*. *marcescens* A | 10 |
| *S*. *marcescens* B | 10 |
| *S*. *enteritidis* A | 10 |
| *S*. *enteritidis B* | 10 |

The quaternary ammonium salt used according to the present invention exhibits a stronger bacteriocidal effect than the conventional disinfectant benzalkonium chloride, while also having equivalent or greater bacteriocidal performance than chlorhexidine digluconate, and it is therefore useful as a disinfectant or the like.

## Claims

1. The use of a
quaternary ammonium salt represented by the following general formula (1): 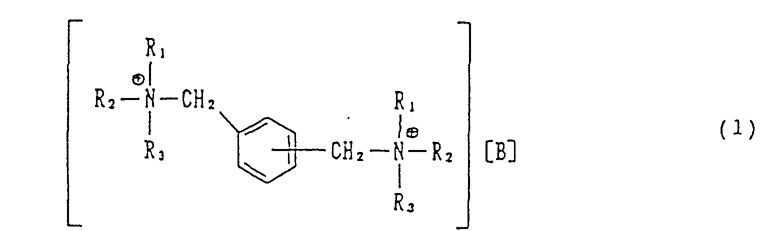 wherein R₁ represents a dodecyl, tridecyl or tetradecyl group, R₂ and R₃ each represent an alkyl group of 1-2 carbon atoms, and [B] represents two monovalent inorganic or organic anion groups or one divalent inorganic or organic anion group as disinfectant.

2. The use according to claim 1, wherein
said inorganic or organic anion group [B] is a sulfate ion, phosphate ion, phosphite ion, hypophosphite ion, nitrate ion, nitrite ion, chlorate ion, chlorite ion, hypochlorite ion or any one of the anion groups represented by the following general formulas (2)-(8).
2[X⁻] (2)
wherein X represents a chlorine atom, bromine atom or iodine atom;
2[R₄COO⁻] (3)
wherein R₄ represents an alkyl group or alkenyl group of 1-7 carbon atoms which may have a hydroxyl group or carbonyl group;
[⁻OCO-R₅-COO⁻] (4)
wherein R₅ represents a direct bond or an alkylene group or alkenylene group of 1-8 carbon atoms which may have a hydroxyl group; 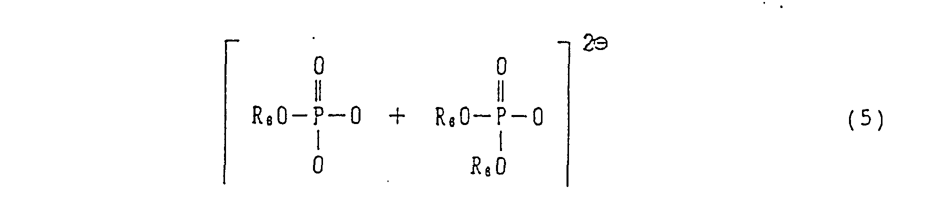 wherein each R₆ represents an alkyl group of 1-8 carbon atoms, and the phosphate ester anion may be a single monoester or diester or a mixture thereof; 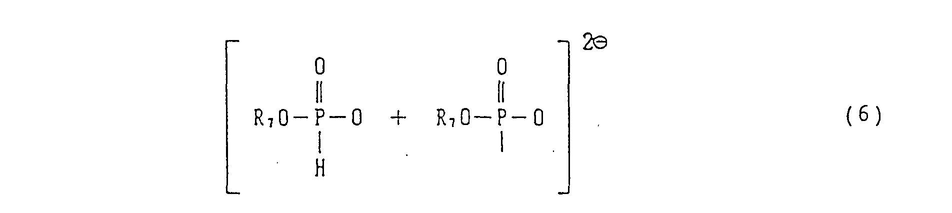 wherein each R₇ represents an alkyl group of 1-8 carbon atoms, and the phosphonate ester anion may be a single monoester or diester or a mixture thereof; 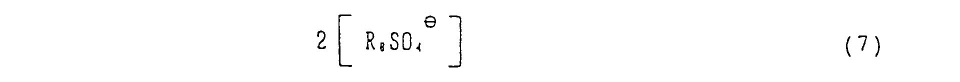 wherein R₈ represents an alkyl group of 1-2 carbon atoms; 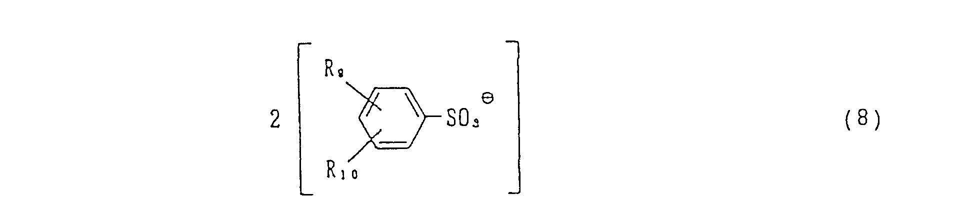 wherein R₉ and R₁₀ each represent a hydrogen atom, methyl group or carboxyl group.

3. The use according to claim 1, wherein
said quaternary ammonium salt is synthesized by reaction between a compound of the following general formula (9) and a compound of the following general formula (10). 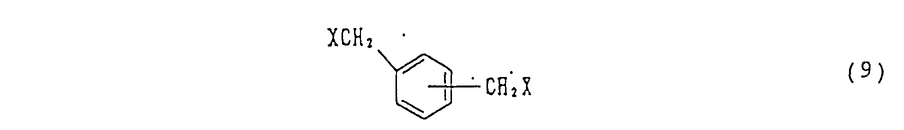 wherein X represents a chlorine atom, bromine atom or iodine atom; 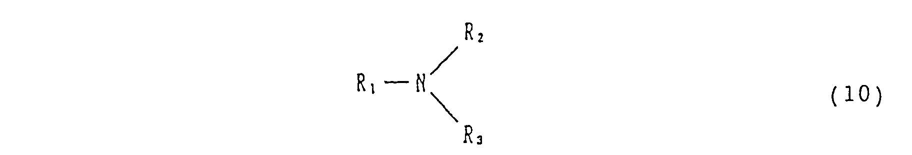 wherein R₁ represents a dodecyl, tridecyl or tetradecyl group, and R₂ and R₃ each represent an alkyl group of 1-2 carbon atoms.

4. The use according to claim 1, wherein
said quaternary ammonium salt is synthesized by reaction between a compound of the following general formula (11) and a compound of the following general formula (12). 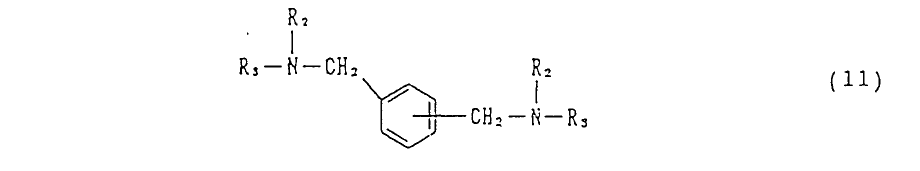 wherein R₂ and R₃ each represent an alkyl group of 1-2 carbon atoms;
R₁-X (12)
wherein R₁ represents a dodecyl, tridecyl or tetradecyl group and X represents a chlorine atom, bromine atom or iodine atom.

5. The use according to claim 1, wherein
said quaternary ammonium salt is synthesized by reaction between a compound of the following general formula (13) and a compound of the following general formula (14). 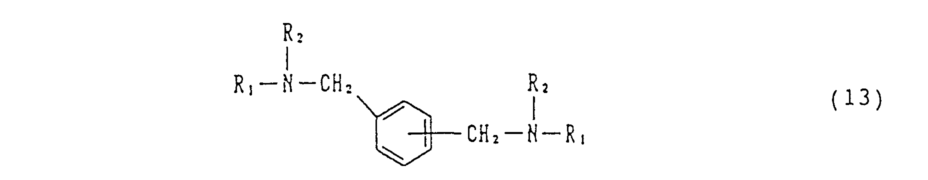 wherein R₁ represents a dodecyl, tridecyl or tetradecyl group and R₂ represents an alkyl group of 1-2 carbon atoms;
R₃-Y (14)
wherein R₃ represents an alkyl group of 1-2 carbon atoms and Y represents a chlorine atom, bromine atom, iodine atom or a group represented by one of the following general formulas (15), (16) or (17): 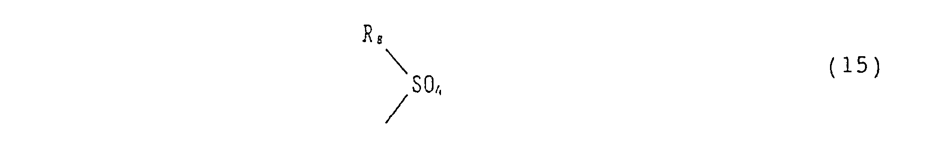 wherein R₈ represents an alkyl group of 1-2 carbon atoms; 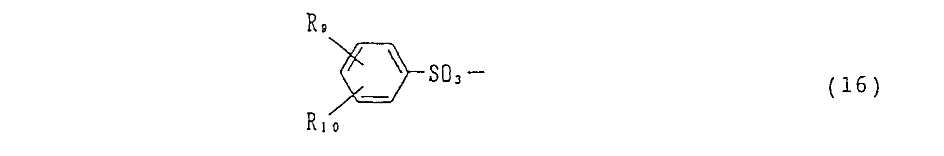 wherein R₉ and R₁₀ each represent a hydrogen atom or methyl group; 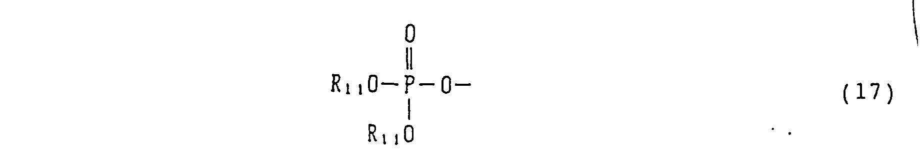 wherein R₁₁ represents an alkyl group of 1-2 carbon atoms.

6. The use according to claim 1, wherein
said quaternary ammonium salt is produced by ion-exchange of a cationic group-containing quaternary ammonium salt compound of the following general formula (18) using sulfuric acid, phosphoric acid, phosphorous acid, hypophosphorous acid, nitric acid, nitrous acid, chloric acid, chlorous acid, hypochlorous acid or a compound of any one of the following general formulas (19), (20), (21), (22) or (23). 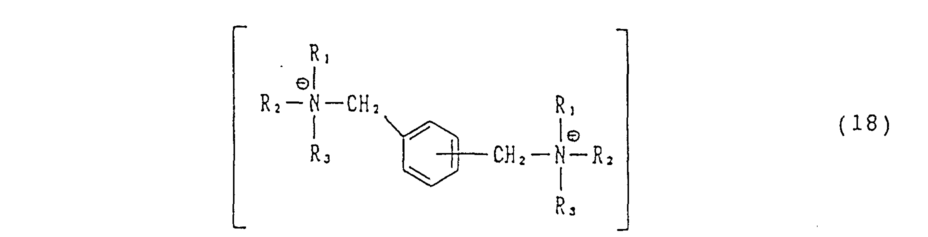 wherein R₁ represents a dodecyl, tridecyl or tetradecyl group, and R₂ and R₃ each represent an alkyl group of 1-2 carbon atoms;
R₄-COOH (19)
wherein R₄ represents an alkyl group or alkenyl group of 1-7 carbon atoms which may have a hydroxyl group or carbonyl group;
HOOC-R₅-COOH (20)
wherein R₅ represents a direct bond or an alkylene group or alkenylene group of 1-8 carbon atoms which may have a hydroxyl group; 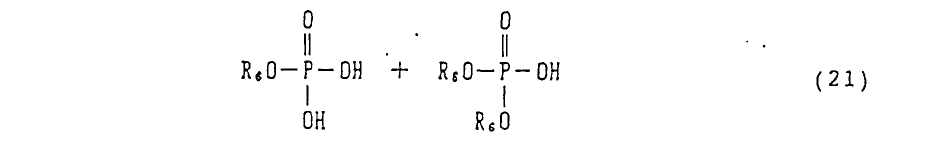 wherein each R₆ represents an alkyl group of 1-8 carbon atoms, and the phosphate ester may be a single monoester or diester or a mixture thereof; 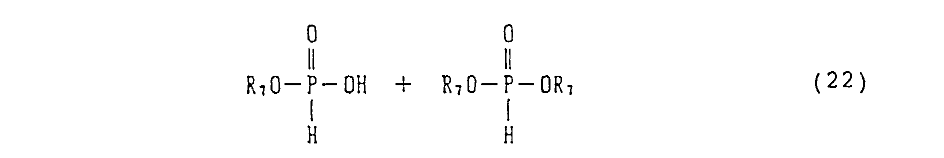 wherein each R₇ represents an alkyl group of 1-8 carbon atoms, and the phosphonate ester may be a single monoester or diester or a mixture thereof; 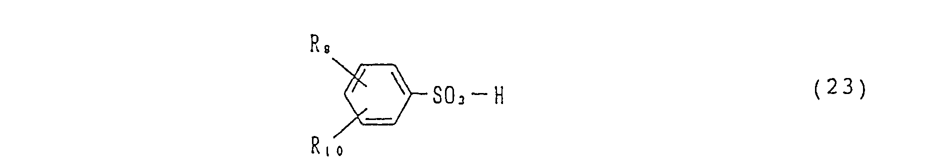 wherein R₉ and R₁₀ each represent a hydrogen atom, methyl group or carboxyl group.

## Patentansprüche

1. Verwendung eines quaternären Ammoniumsalzes mit der allgemeinen Formel (1) 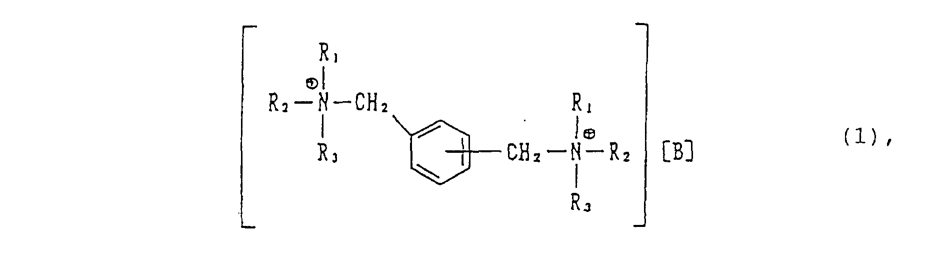 worin R₁ einen Dodecyl-, Tridecyl- oder Tetradecylrest bedeutet, R₂ und R₃ jeweils einen Alkylrest mit 1 bis 2 Kohlenstoffatomen bedeuten und [B] zwei einwertige anorganische bzw. organische anionische Reste oder einen zweiwertigen anorganischen bzw. organischen anionischen Rest bedeutet, als Desinfektionsmittel.

2. Verwendung nach Anspruch 1, wobei der anorganische bzw. organische anionische Rest [B] ein Sulfat-, Phosphat-, Phosphit-, Phosphinat-, Nitrat-, Nitrit-, Chlorat-, Chlorit- und Hypochlorition oder einen der anionischen Reste bedeutet, welche die allgemeine Formeln (2) bis (8) besitzen:
2[X⁻] (2),
worin X ein Chlor-, Brom- oder Iodatom bedeutet,
2[R₄COO⁻] (3),
worin R₄ einen Alkylrest oder einen Alkenylrest mit 1 bis 7 Kohlenstoffatomen, der eine Hydroxylgruppe oder Carbonylgruppe tragen kann, bedeutet,
[⁻OCO-R₅-COO⁻] (4),
worin R₅ eine direkte Bindung oder einen Alkylenrest bzw. Alkenylenrest mit 1 bis 8 Kohlenstoffatomen, der eine Hydroxylgruppe tragen kann, bedeutet, 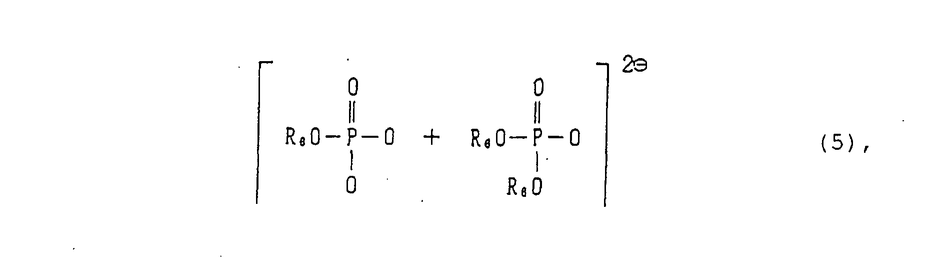 worin R₆ jeweils einen Alkylrest mit 1 bis 8 Kohlenstoffatomen bedeutet und das Phosphorsäureesteranion ein einziger Monoester bzw. Diester oder ein Gemisch davon sein kann, 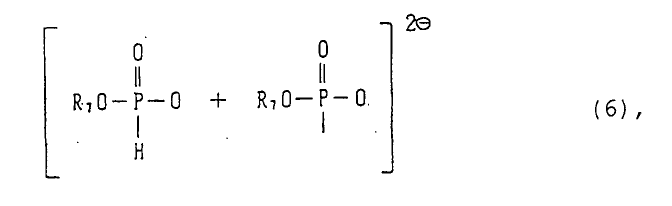 worin R₇ jeweils einen Alkylrest mit 1 bis 8 Kohlenstoffatomen bedeutet und das Phosphonsäureesteranion ein einziger Monoester bzw. Diester oder ein Gemisch davon sein kann,
2[R₈SO₄ ^{⊖}] (7),
worin R₈ einen Alkylrest mit 1 bis 2 Kohlenstoffatomen bedeutet, und 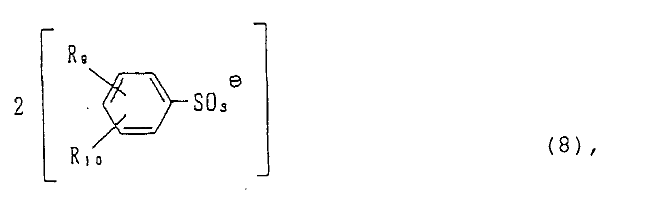 worin R₉ und R₁₀ jeweils ein Wasserstoffatom und einen Methylrest oder Carboxylrest bedeuten.

3. Verwendung nach Anspruch 1, wobei das quaternäre Ammoniumsalz durch Umsetzung einer Verbindung mit der allgemeinen Formel (9) mit einer Verbindung mit der allgemeinen Formel (10) hergestellt ist: 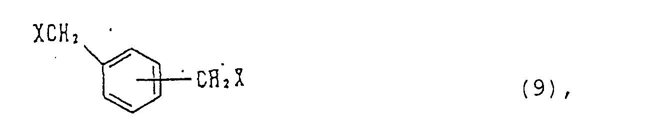 worin X ein Chlor-, Brom- oder Iodatom bedeutet, 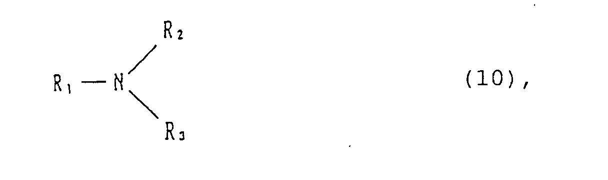 worin R₁ einen Dodecyl-, Tridecyl- oder Tetradecylrest bedeutet und R₂ und R₃ jeweils einen Alkylrest mit 1 bis 2 Kohlenstoffatomen bedeuten.

4. Verwendung nach Anspruch 1, wobei das quaternäre Ammoniumsalz durch Umsetzung einer Verbindung mit der allgemeinen Formel (11) mit einer Verbindung mit der allgemeinen Formel (12) hergestellt ist: 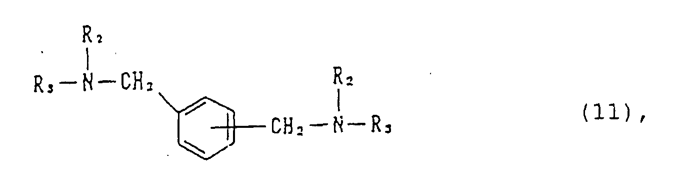 worin R₂ und R₃ jeweils einen Alkylrest mit 1 bis 2 Kohlenstoffatomen bedeuten,
R₁-X (12),
worin R₁ einen Dodecyl-, Tridecyl- oder Tetradecylrest und X ein Chlor-, Brom- oder Iodatom bedeutet.

5. Verwendung nach Anspruch 1, wobei das quaternäre Ammoniumsalz durch Umsetzung einer Verbindung mit der allgemeinen Formel (13) mit einer Verbindung mit der allgemeinen Formel (14) hergestellt ist: 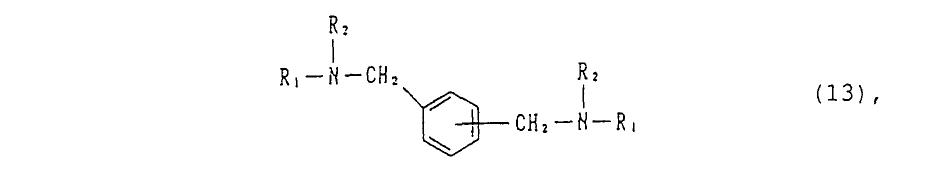 worin R₁ einen Dodecyl-, Tridecyl- oder Tetradecylrest und R₂ einen Alkylrest mit 1 bis 2 Kohlenstoffatomen bedeutet, und
R₃-Y (14),
worin R₃ einen Alkylrest mit 1 bis 2 Kohlenstoffatomen und Y ein Chlor-, Brom- und Iodatom oder einen Rest mit einer der allgemeinen Formeln (15), (16) oder (17) bedeutet: 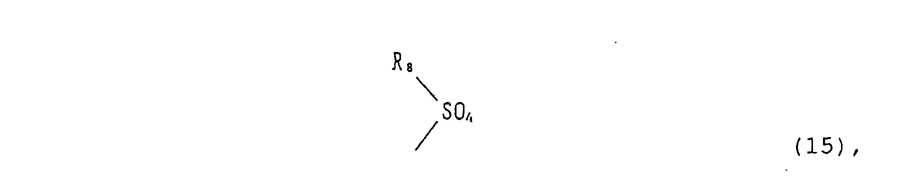 worin R₈ einen Alkylrest mit 1 bis 2 Kohlenstoffatomen bedeutet, 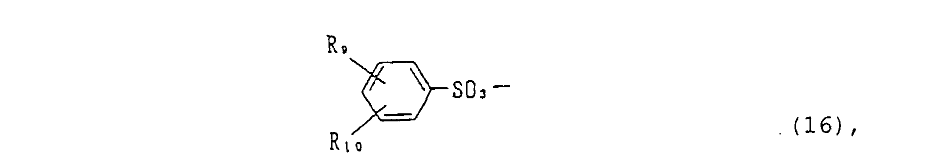 worin R₉ und R₁₀ jeweils ein Wasserstoffatom oder einen Methylrest bedeuten, und 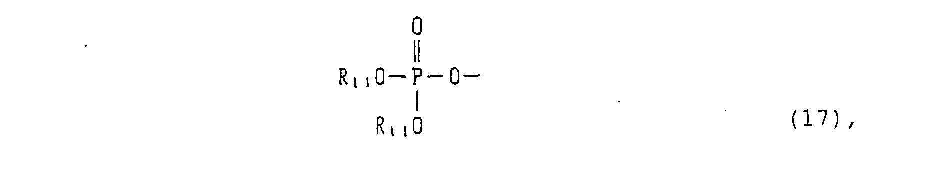 worin R₁₁ einen Alkylrest mit 1 bis 2 Kohlenstoffatomen bedeutet.

6. Verwendung nach Anspruch 1, wobei das quaternäre Ammoniumsalz durch Ionenaustausch einer kationische Reste enthaltenden quaternären Ammoniumsalzverbindung mit der allgemeinen Formel (18) unter Verwendung von Schwefelsäure, Phosphorsäure, phosphoriger Säure, Phosphinsäure, Salpetersäure, salpetriger Säure, ' Chlorsäure, chloriger Säure und Hypochlorsäure oder einer Verbindung mit einer der allgemeinen Formeln (19), (20), (21), (22) oder (23) hergestellt ist: 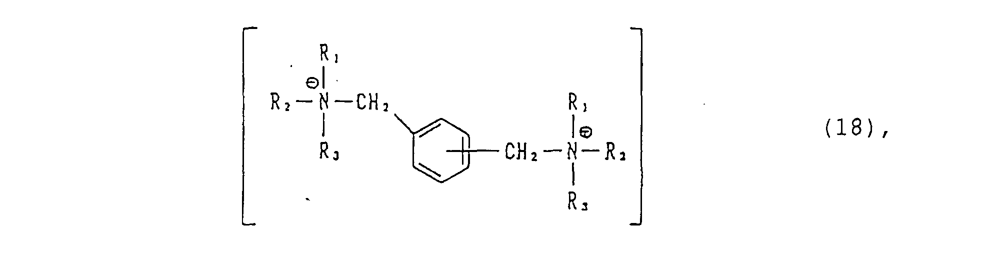 worin R₁ einen Dodecyl-, Tridecyl- oder Tetradecylrest bedeutet und R₂ und R₃ jeweils einen Alkylrest mit 1 bis 2 Kohlenstoffatomen bedeuten,
R₄-COOH (19),
worin R₄ einen Alkylrest oder einen Alkenylrest mit 1 bis 7 Kohlenstoffatomen, der eine Hydroxylgruppe oder Carbonylgruppe tragen kann, bedeutet,
HOOC-R₅-COOH (20),
worin R₅ eine direkte Bindung oder einen Alkylenrest bzw. Alkenylenrest mit 1 bis 8 Kohlenstoffatomen, der eine Hydroxylgruppe tragen kann, bedeutet, 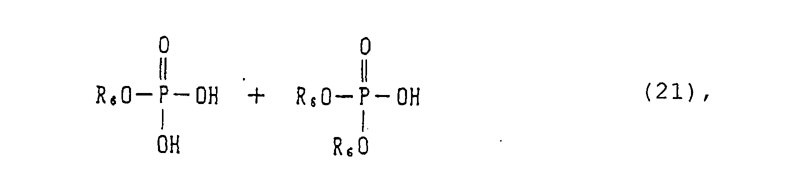 worin R₆ jeweils einen Alkylrest mit 1 bis 8 Kohlenstoffatomen bedeutet und der Phosphorsäureester ein einziger Monoester bzw. Diester oder ein Gemisch davon sein kann, 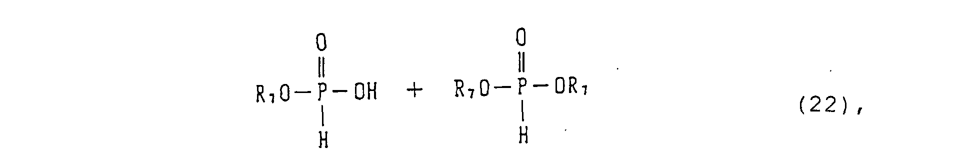 worin R₇ jeweils einen Alkylrest mit 1 bis 8 Kohlenstoffatomen bedeutet und der Phosphonsäureester ein einziger Monoester bzw. Diester oder ein Gemisch davon sein kann, und 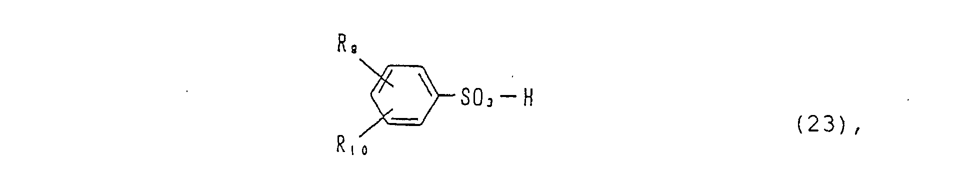 worin R₉ und R₁₀ jeweils ein Wasserstoffatom und einen Methylrest oder Carboxylrest bedeuten.

## Revendications

1. Utilisation d'un sel d'ammonium quaternaire, représenté par la formule générale 1 ci-après : 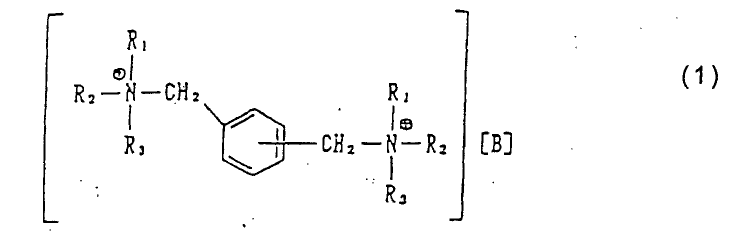 Dans laquelle R₁ représente un groupe dodécyle, tridécyle ou tétradécyle, R₂ et R₃ représentent chacun un groupe alkyle ayant 1-2 atomes de carbone, et [B] représente deux groupes anioniques organiques ou inorganiques monovalents ou un groupe anionique inorganique ou organique divalent, en tant que désinfectant.

2. Utilisation selon la revendication 1, dans laquelle ledit groupe anionique inorganique ou organique [B] est un ion sulfate, un ion phosphate, un ion phosphite, un ion hypophosphite, un ion nitrate, un ion nitrite, un ion chlorate, un ion chlorite, un ion hypochlorite, ou l'un quelconque des groupes anioniques représentés par les formules générales (2) à (8) ci-après :
2[X⁻] (2)
où X représente un atome de chlore, un atome de brome ou un atome d'iode ;
2[R₄COO⁻] (3)
où R₄ représente un groupe alkyle ou alcényle ayant de 1 à 7 atomes de carbone, qui peut comporter un groupe hydroxyle ou un groupe carbonyle ;
[⁻OCO-R₅-COO⁻] (4)
où R₅ représente une liaison directe ou un groupe alkylène ou un groupe alcénylène ayant de 1 à 8 atomes de carbone, qui peut comporter un groupe hydroxyle ; 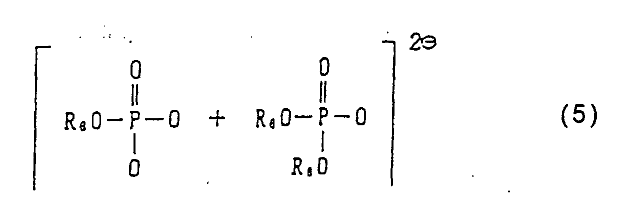 où chaque R₆ représente un groupe alkyle ayant de 1 à 8 atomes de carbone, et l'anion ester phosphate peut être un monoester unique, ou un diester unique ou un mélange de ces derniers ; 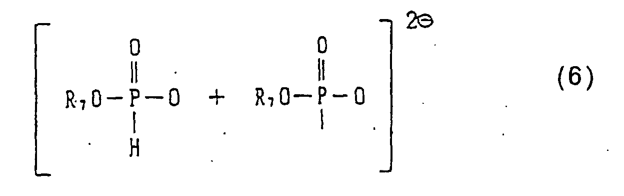 Où chaque R₇ représente un groupe alkyle ayant de 1 à 8 atomes de carbone, et l'anion esterphosphonate peut être un monoester unique ou un diester unique, ou un mélange de ces derniers ; 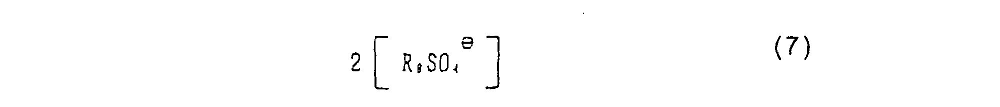 Où R₈ représente un groupe alkyle ayant 1-2 atomes de carbone ; 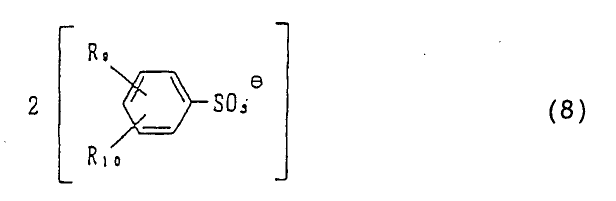 Où R₉ et R₁₀ représentent chacun un atome d'hydrogène, un groupe méthyle ou un groupe carboxyle.

3. Utilisation selon la revendication 1, dans laquelle ledit sel d'ammonium quaternaire est synthétisé par réaction entre un composé ayant la formule générale (9) ci-après et un composé ayant la formule générale (10) ci-après 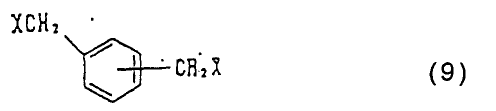 où X représente un atome de chlore, un atome de brome ou un atome d'iode ; 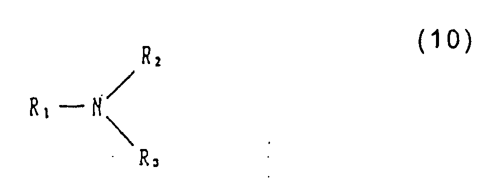 où R₁ représente un groupe dodécyle, tridécyle ou tétradécyle, et R₂ et R₃ représentent chacun un groupe alkyle ayant 1-2 atomes de carbone.

4. Utilisation selon la revendication 1, dans laquelle ledit sel d'ammonium quaternaire est synthétisé par réaction entre un composé ayant la formule générale (11) ci-après et un composé ayant la formule générale (12) ci-après 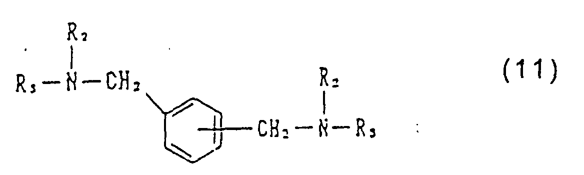 où R₂ et R₃ représentent chacun un groupe alkyle ayant 1-2 atomes de carbone,
R₁-X (12)
Où R₁ représente un groupe dodécyle, tridécyle ou tétradécyle, et X représente un atome de chlore, un atome de brome ou un atome d'iode.

5. Utilisation selon la revendication 1, dans laquelle ledit sel d'ammonium quaternaire est synthétisé par réaction entre un composé ayant la formule générale (13) ci-après et un composé ayant la formule générale (14) ci-après : 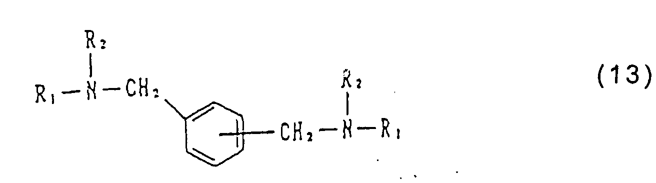 Où R₁ représente un groupe dodécyle, tridécyle ou tétradécyle, et R₂ représente un groupe alkyle ayant 1-2 atomes de carbone ;
R₃-Y (14)
Où R₃ représente un groupe alkyle ayant 1-2 atomes de carbone, et Y représente un atome de chlore, un atome de brome, un atome d'iode, ou un groupe représenté par l'une des formules générales (15), (16), ou (17) ci-après : 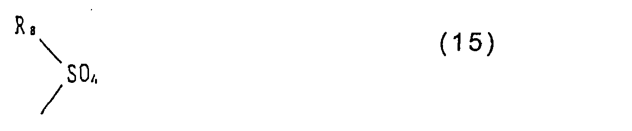 Où R₈ représente un groupe alkyle ayant 1-2 atomes de carbone : 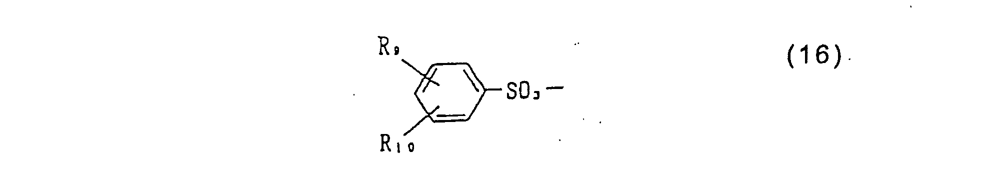 Où R₉ et R₁₀ représentent chacun un atome d'hydrogène ou un groupe méthyle ; 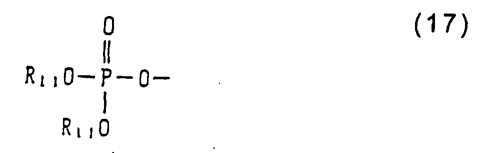 Où R₁₁ représente un groupe alkyle ayant 1-2 atomes de carbone.

6. Utilisation selon la revendication 1, dans laquelle ledit sel d'ammonium quaternaire est produit par échange d'ions d'un composé sel d'ammonium quaternaire contenant un groupe cationique ayant la formule générale 18 ci-après par utilisation d'acide sulfurique, d'acide phosphorique, d'acide phosphoreux, d'acide hypophosphoreux, d'acide nitrique, d'acide nitreux, d'acide chlorique, d'acide chloreux, d'acide hypochloreux ou d'un composé ayant l'une quelconque des formules générales (19), (20), (21), (22) ou (23) ci-après 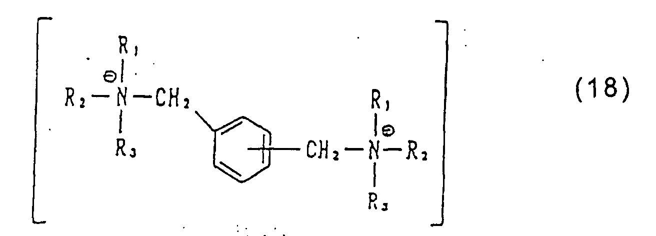 Où R₁ représente un groupe dodécyle, tridécyle ou tétradécyle, et R₂ et R₃ représentent chacun un groupe alkyle ayant 1-2 atomes de carbone ;
R₄-COOH (19)
Où R₄ représente un groupe alkyle ou un groupe alcényle ayant de 1 à 7 atomes de carbone, qui peut comporter un groupe hydroxyle ou un groupe carbonyle ;
HOOC-R₅-COOH (20)
Où R₅ représente une liaison directe ou un groupe alkylène ou un groupe alcénylène ayant de 1 à 8 atomes de carbone, qui peut comporter un groupe hydroxyle ; 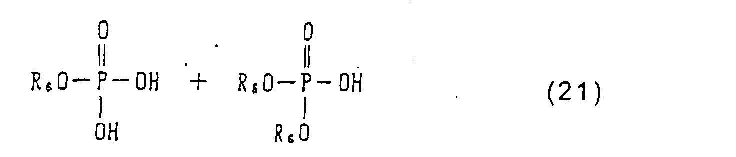 Où chaque R₆ représente un groupe alkyle ayant de 1 à 8 atomes de carbone, et l'ester phosphate peut être un monoester unique ou un diester unique, ou un mélange de ces derniers ; 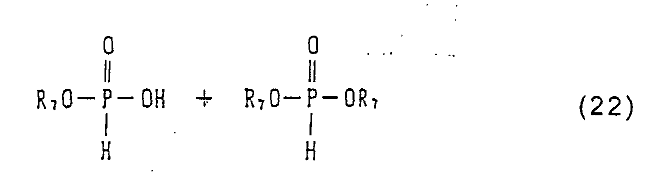 Où chaque R₇ représente un groupe alkyle ayant de 1 à 8 atomes de carbone, et l'ester phosphonate peut être un monoester unique ou un diester unique, ou un mélange de ces derniers ; 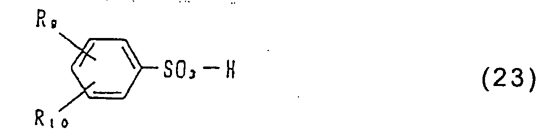 Où R₉ et R₁₀ représentent chacun un atome d'hydrogène, un groupe méthyle ou un groupe carboxyle.
